# EUROPEAN PATENT APPLICATION

(11) **EP 3 928 775 A1**
(43) Date of publication of application: **29.12.2021**
(21) Application number: 20305698.1
(22) Date of filing: 24.06.2020
(51) Int. Cl.: A61K 31/135, A61K 31/137, A61K 31/138, A61K 31/15, A61K 31/165, A61K 31/232, A61K 31/343, A61K 31/381, A61K 31/4406, A61K 31/443, A61K 31/445, A61K 31/454, A61K 31/4965, A61K 31/497, A61K 31/55, A61K 31/5513, A61P 31/14

(54) **USE OF ANTIDEPRESSANT AGENTS FOR TREATING THE COVID-19 DISEASE**

(71) Applicant: ASSISTANCE PUBLIQUE, HOPITAUX DE PARIS, 75004 Paris (FR)
(72) Inventor: HOERTEL, Nicolas, 92130 Issy-les-Moulineaux (FR); LIMOSIN, Frédéric, 75015 Paris (FR); SANCHEZ RICO, Marina Lucia, 92130 Issy-les-Moulineaux (FR)
(74) Representative: Regimbeau

(57) **Abstract**

The present invention relates to the therapeutic uses of antidepressant compounds such as SSRIs, SNRIs and a2-antagonist antidepressants for lowering the risk of death and/or intubation in patient suffering from a viral infection caused by at least one betacoronavirus, in particular by the SARS-CoV-2 (Severe Acute Respiratory Syndrome Coronavirus 2).

## Description

### SUMMARY OF THE INVENTION

The present invention relates to the therapeutic uses of antidepressant compounds such as SSRIs, SNRIs and α2-antagonist antidepressants for lowering the risk of death and/or intubation in patient suffering from a viral infection caused by at least one betacoronavirus, in particular by the SARS-CoV-2 (Severe Acute Respiratory Syndrome Coronavirus 2).

### BACKGROUND OF THE INVENTION

Global spread of the novel coronavirus SARS-CoV-2, the causative agent of coronavirus disease 2019 (Covid-19), has created an unprecedented infectious disease crisis worldwide. In the current absence of a vaccine or medications with published evidence-based clinical efficacy,^{1,2} the search for an effective treatment for patients with Covid-19 among all available medications is urgently needed.^{3,4}

Both Covid-19 and severe acute respiratory syndrome (SARS) are characterized by an excessive inflammatory response⁵ and, for Covid-19, viral load may be correlated with the worsening of symptoms.⁶

Prior work suggests a significant reduction of overactive inflammatory processes following antidepressant treatment among people with major depressive disorder.^{7,8} Specifically, a recent meta-analysis⁷ of studies conducted in this population supports that antidepressants may decrease plasma levels of the pro-inflammatory cytokines IL-6, TNF-α, and CCL-2, which are involved in the pathogenesis of severe Covid-19⁹. Most studies in that meta-analysis included selective serotonin reuptake inhibitors (SSRIs) or serotonin-norepinephrine reuptake inhibitors medications (SNRIs), and prior work suggested that SSRIs could more potently inhibit microglial TNF-α and NO production than SNRIs, possibly though cAMP signaling regulation.¹⁰ Furthermore, a recent in-vitro study suggests antiviral effects of the serotonin reuptake inhibitor fluoxetine on SARS-CoV-2. Interestingly, this effect has not be observed for other serotonin reuptake inhibitors, including escitalopram and paroxetine.¹¹

SARS-CoV-2 belongs to the species *Coronavirus,* in the genus *Betacoronavirus* and family *Coronaviridae.* Coronaviruses are enveloped viruses with a helically symmetrical capsid. They have a single-stranded, positive-sense RNA genome and are capable of infecting cells in birds and mammals. The morphology of the virions is typical, with a halo of protein protuberances ('Spike') which gave them their name of 'crown virus'. Among the four genera of coronaviruses, the Betacoronavirus genus (β-CoVs or Beta-CoVs), comprising virus infecting animals and/or humans, is subdivided into four lineages designated as A, B, C and D:
- Lineage A also designated as subgenus *Embecovirus* includes HCoV-OC43 and HCoV-HKU1, virus able to infect various species;
- Lineage B also designated as subgenus *Sarbecovirus* includes SARS-CoV-1, SARS-CoV-2, and Bat SL-CoV-WIV1;
- Lineage C also designated as subgenus *Merbecovirus* includes Tylonycteris bat coronavirus HKU4 (BtCoV-HKU4), Pipistrellus bat coronavirus HKU5 (BtCoV-HKU5), and MERS-CoV, able to infect notably camels and humans;
- Lineage D also designated as subgenus *Nobecovirus* includes Rousettus bat coronavirus HKU9 (BtCoV-HKU9).

The Betacoronaviruses of the greatest clinical importance concerning humans are:
- OC43 and HKU1 of the A lineage,
- SARS-CoV-1 and SARS-CoV-2 of the B lineage, and
- MERS-CoV of the C lineage.

In humans, coronavirus infections can cause respiratory pathologies associated with symptoms similar to the common cold, bronchiolitis and more serious diseases such as the Severe Acute Respiratory Syndrome caused by SARS-CoV-1, which generated an epidemic in 2003, and the Middle Eastern Respiratory Syndrome caused by MERS-CoV, which generated an epidemic in 2012. SARS-CoV-2 is the betacoronavirus causing the coronavirus epidemic of 2019-2020, generating the form of pneumonia known as coronavirus disease 2019 or COVID-19. According to the World Health Organization (WHO), by April 5, 2020, 1 133 758 cases of COVID-19 have been confirmed and 62 784 patients have died worldwide. This epidemic was declared a public health emergency of international concern by WHO on January 30, 2020.

Symptoms of infection with SARS-CoV-2 are roughly similar to those of seasonal influenza infections: they include fever, fatigue, dry cough, shortness of breath, difficult breathing, pneumonia, renal failure, and may lead to death in severe cases.

The severity of clinical signs requires that approximately 20% of patients remain in hospital and 5% require admission to intensive care. WHO estimates the mortality rate to be around 2% (data of February 2020). However, this case-fatality rate remains uncertain, due to the difficulty in estimating the number of confirmed cases and deaths directly attributable to SARS-CoV-2 infection in the field. The most serious forms are observed in people who are vulnerable because of their age (over 70) or associated diseases such as hypertension, diabetes and/or coronary heart disease.

The majority of treatments currently received by COVID-19 patients are primarily aimed at alleviating the symptoms of fever, cough and dyspnea in order to promote spontaneous recovery. Yet, no efficient treatment has ever been proposed to prevent intubation or death of the patient once they are in a severe stage of the disease.

It is of the most importance to identify therapeutic compounds for treating the COVID-19 disease in order to prevent intubation or death of severely infected patient. The present invention fulfills this need.

### DESCRIPTION OF THE INVENTION

As a matter of fact, the present inventors discovered that the use of certain antidepressant compounds, and specifically SSRIs and SNRIs, significantly lower the risk of a composite endpoint of intubation or death in COVID19 patients. By performing time-to-event analyses adjusted for numerous potential confounders (including sex, age, obesity, self-reported smoking status, any medical condition, any medication prescribed according to compassionate use or as part of clinical trials, clinical and biological severity of Covid-19 at admission, any current mood or anxiety disorder, any other current psychiatric disorder, any prescribed benzodiazepine or Z-drug, mood stabilizer, or antipsychotic medication), they showed that there is a strong association between the use of SSRIs, SNRIs, and α2-antagonist antidepressants with respiratory failure among adult patients who have been admitted to medical centers with Covid-19.

More precisely, the results presented below show that among COVID-19 patients receiving antidepressants, SSRIs, SNRIs, tricyclic antidepressants, tetracyclic antidepressants, and α2-antagonist antidepressants, the primary endpoint of death or intubation occurred in 143 patients (31.1%), 75 patients (29.2%), 18 patients (25.4%), 19 patients (32.2%), 43 patients (45.7%) and 12 patients (27.3%), respectively, whereas 1,188 (17.3%) non-exposed patients had this outcome. When adjusting for the older age and the greater medical severity of patients receiving antidepressants, the primary multivariable analysis with inverse probability weighting according to the propensity score showed a significant negative association of the composite endpoint with exposure to any antidepressant (HR, 0.64; 95% CI, 0.51 to 0.80, p<0.001), SSRIs (HR, 0.56; 95% CI, 0.42 to 0.75, p<0.001), and SNRIs (HR, 0.57; 95% CI, 0.34 to 0.96, p=0.034). Exposure to escitalopram (HR, 0.61; 95% CI, 0.40 to 0.92, p=0.018), fluoxetine (HR, 0.32; 95% CI, 0.14 to 0.73, p=0.007), or venlafaxine (HR, 0.47; 95% CI, 0.24 to 0.91, p=0.025) was significantly associated with a reduced risk of intubation or death. In sensitivity analyses, multivariable Cox regression models in the full sample yielded similar results, as did univariate Cox regression models in the matched analytic samples, except for exposure to SNRIs which was not significant (HR, 0.85; 95% CI, 0.32 to 1.05, p=0.074), and exposures to paroxetine (HR, 0.62; 95% CI, 0.40 to 0.96, p=0.034) and mirtazapine (HR, 0.52; 95% CI, 0.27 to 0.99, p=0.046), which were significantly associated with a reduced risk of death or intubation in these analyses.

### Definitions

In the context of the invention, the term "betacoronavirus" designates any virus belonging to the Betacoronavirus genus (β-CoVs or Beta-CoVs), in particular any betacoronavirus belonging to one of the four lineages designated as A, B, C and D. It designates a betacoronavirus infecting animals and/or humans. In particular, this designation includes the betacoronaviruses infecting human organisms chosen among OC43, HKU1, SARS-CoV-1, SARS-CoV-2 and MERS-CoV.

The term "viral infection due to at least one betacoronavirus" designates the fact that host cells of an organism have been infected by at least one betacoronavirus, the whole organism being said to have a viral infection.

A betacoronavirus infection in humans is usually diagnosed by a healthcare professional, based on observation of the infected patient's symptoms. Additional biological tests may be required to confirm the diagnosis: blood and/or sputum and/or bronchoalveolar fluid tests. Infection by a betacoronavirus can be established, for example, by molecular biological detection and/or viral titration of respiratory specimens, or by assaying blood for antibodies specific for said betacoronavirus. Conventional diagnostic methods comprise techniques of molecular biology such as PCR, which is well known to the person in the field.

As used herein, the term "treatment" refers to fighting the betacoronavirus infection in a human or animal organism. By administering the agent of the invention, the symptoms associated with the betacoronovirus infection (respiratory syndrome, etc.) will be reduced, or the severity of the disease will decrease. It is also possible, with the treatment of the invention, to prevent the worsening of the disease.

In the context of the invention, the terms "COVID-19 disease" mean the disease linked to the infection with the SARS-CoV-2 (Severe Acute Respiratory Syndrome Coronavirus 2).

In the context of the invention, a "symptomatic COVID-19 disease" is characterized by a patient who shows at least one symptom of the COVID-19 disease. The most common symptoms of the COVID-19 disease are fever, muscle aches, headaches, fatigue, loss of taste and smell and respiratory symptoms such as a dry cough, difficulty breathing and a lack of oxygen. A symptomatic disease is in contrast to an asymptomatic disease which is characterized by a patient who is a carrier for a disease or infection but experiences no symptoms.

In particular, for the betacoronavirus disease (such as COVID-19), the following forms are usually observed:
- an **"asymptomatic form"** wherein the subject is a carrier of at least on betacoronavirus but shows no symptom.
- a **"mild COVID form"** wherein the subject shows the first symptoms (or signs) of a COVID disease, as listed above. Mild COVID symptoms (such as COVID-19) comprise e.g., mild dry cough, mild muscle pain, mild headache, mild fever, mild fatigue, loss of taste or smell.
- a **"strong COVID form"** wherein the subject shows strong respiratory symptoms such as difficulty breathing, lack of oxygen; other stronger symptoms such as fever, dry cough, aches and pains, nasal congestion, strong headache, conjunctivitis, sore throat, skin rash, discoloration of fingers or feet, or any combination thereof; as well as a deterioration of the general state of health with frequent diarrhoea, but also liver or urinary disorders, dizziness or neuromuscular problems; some of these symptoms may require hospitalisation. Most patients have an abnormal chest X-ray or CT scan within the first few days of illness, even in the absence of respiratory signs.
- A **"severe COVID form"** or **"critical COVID form"** or **"aggressive COVID form"** wherein the subject has life-threatening symptoms (or signs) of COVID (e.g. COVID-19), comprising at least one selected from (but not limited to) : respiratory distress, lung disorders, liver disorders, kidney disorders, neuromuscular disorders, brain disorders, etc, that requires hospitalisation and/or intensive care (in intensive care units (ICU)).

In the context of the invention, the "severe symptomatic COVID-19 disease" is characterized by severe symptoms of the COVID-19 disease, in particular respiratory symptoms. Severe symptoms are acute respiratory distress syndrome that requires hospitalization of the patient in any unit, and especially in the intensive care unit (ICU) in case of critical infection. In the context of the invention, the abbreviation "ICU" refers to an intensive care unit, a special department of a hospital or health care facility that provides intensive treatment medicine.

In the context of the invention, "COVID-19 patients" are human patients infected with the SARS-CoV-2 (Severe Acute Respiratory Syndrome Coronavirus 2).

As used herein, the term "treatment of the invention" or "agent of the invention" designates any 5 HT-stimulating or blocking agent, that is able to act directly or indirectly on a serotonin receptor (also known as 5 HT receptor). By "5-hydroxytryptamine receptor (5-HT)-stimulating agent", or "5-HT-stimulating agent" or "5 HT-stimulating agent", it is meant herein an active agent capable of stimulating, either directly or indirectly, the activity of a 5-hydroxytryptamine receptor, or in other words, capable of mimicking or enhancing the effects that are usually exerted by the natural ligand of said receptor (i.e. serotonin). By "5-hydroxytryptamine receptor (5-HT)-blocking agent", or "5-HT-blocking agent" or "5 HT-blocking agent", it is meant herein an active agent capable of blocking or inhibiting, either directly or indirectly, the activity of a 5-hydroxytryptamine receptor, or in other words, capable of blocking or preventing the effects that are usually exerted by the natural ligand of said receptor (i.e. serotonin).

In a preferred embodiment, said agent is a 5 HT1 R-stimulating or blocking agent, i.e., an agent that is able to stimulate or block, directly or indirectly, a 5 HT1 receptor such as 5 HT1 A, 5 HT1 B, 5 HT1 D, 5 HT1 E, 5 HT1 F receptor.

In another preferred embodiment, said agent is a 5 HT2 R-stimulating or blocking agent, that is, an agent able to stimulate or block, directly or indirectly, a 5 HT2 receptor such as 5 HT2 A, 5 HT2 B, or 5 HT2 C receptor.

In a more preferred embodiment, the agent of the invention is a "5-hydroxytryptamine 1B receptor (5-HT1 BR)-stimulating agent", or "5-HT1 BR-stimulating agent" or "5 HT1 BR-stimulating agent", that is, an active agent capable of stimulating, either directly or indirectly, the activity of the 5-hydroxytryptamine 1B receptor, or in other words, capable of mimicking or enhancing the effects that are usually exerted by the natural ligand of said receptor (i.e. serotonin).

In all cases, a "direct" stimulation or blocking requires the binding of the agent to said receptor, while an "indirect" stimulation or blocking does not involve the binding of said agent to said receptor (i.e. it acts on the receptor via another mechanism of action).The capacity of a candidate agent to activate or block said receptor can be assessed by methods well-known in the art, for example by overexpressing the 5-HT receptor (in particular the 5-HT1 BR) in cells and measuring intracellular cAMP production before and after contact of the recombinant cells with said candidate agent. Other methods for measuring the activity of the 5-HT receptor (in particular the 5-HT1 BR) have been described in the art, and include, among others, a reverse phase high performance liquid chromatography coupled to an electrochemical detector (HPLC-ED) using an amperometric detector. In the context of the present invention, said agent can be selective for the 5-HT receptor (in particular the 5-hydroxytryptamine 1B receptor), or may act not only on the 5-HT receptor (in particular the 5-hydroxytryptamine 1B receptor) but also on other receptors (such as other 5-HT receptors).

By "derivative", it is meant herein a compound that is directly derived from a chemical compound of interest (i.e. 5-HT -stimulating or blocking agent) and is structurally similar though non-identical to said compound, and which retains the same biological activity and/or physico-chemical properties.

By "analog", or "functional analog", it is meant herein a compound that is not directly derived from a chemical compound of interest and is thus structurally different, but exhibits the same biological activity and/or physico-chemical properties, such as isosters.

"Derivatives" and "analogs" of the agents according to the invention encompass herein compounds that retain the 5-HT-stimulating or blocking activity as defined above.

By "isomer", it is meant herein a compound having the same chemical formula as a compound of interest, but a different chemical structure. This term encompasses structural isomers and stereoisomers. Should the isomer of the invention be a stereoisomer, the individual stereoisomers (enantiomers and diastereoisomers) and mixtures thereof are included within the scope of the invention. Some of the compounds according to the invention may exist in tautomeric forms (a type of structural isomer), which are also included within the scope of the invention.

By "metabolite" as used herein, it is meant any compound that is an intermediate and/or a product of metabolism. A metabolite from a chemical compound is usually formed as part of the natural biochemical process of degrading and eliminating the compound of interest in a subject to which it is administered. Examples of metabolites of antidepressant agents according to the invention are provided further below.

The term "solvate" according to the invention should be understood as meaning any form of the active agent in accordance with the invention (i.e. 5-HT-stimulating or blocking agent), in which said compound is linked through non-covalent interactions to another molecule (normally a polar solvent), including especially hydrates and alcoholates, such as methanolate. Methods of solvation are well-known in the art.

By "clathrate", it is meant herein a chemical substance consisting of a lattice or cage that entraps or contains a second type of molecule/compound of interest, and which can be used to increase the stability and solubility in water of the molecule/compound of interest. Clathrates are typically polymeric.

The term "polymorphs" means herein different crystalline forms of a compound of interest in which molecules have different arrangements and/or different molecular conformation. It includes crystalline liquid form or crystalline solid form of a compound of interest. Hydrates and clathrates can be polymorphs.

By "co-crystal", it is meant herein a crystalline structure composed of at least two components, where the components may be atoms, ions or molecules. Solvates and clathrates may be co-crystals in certain conditions.

The term "pharmaceutically acceptable salt" is intended to mean, in the framework of the present invention, a salt of a compound which is pharmaceutically acceptable, as defined above, and which possesses the pharmacological activity of the corresponding compound.

The pharmaceutically acceptable salts comprise:
1) acid addition salts formed with inorganic acids such as hydrochloric, hydrobromic, sulfuric, nitric and phosphoric acid and the like; or formed with organic acids such as acetic, benzenesulfonic, fumaric, glucoheptonic, gluconic, glutamic, glycolic, hydroxynaphtoic, 2-hydroxyethanesulfonic, lactic, maleic, malic, mandelic, methanesulfonic, muconic, 2-naphtalenesulfonic, propionic, succinic, dibenzoyl-L-tartaric, tartaric, p-toluenesulfonic, trimethylacetic, and trifluoroacetic acid and the like, and
2) base addition salts formed when an acid proton present in the compound is either replaced by a metal ion, such as an alkali metal ion, an alkaline-earth metal ion, or an aluminium ion; or coordinated with an organic or inorganic base. Acceptable organic bases comprise diethanolamine, ethanolamine, N-methylglucamine, triethanolamine, tromethamine and the like. Acceptable inorganic bases comprise aluminium hydroxide, calcium hydroxide, potassium hydroxide, sodium carbonate and sodium hydroxide.

In the context of the present invention, the pharmaceutically acceptable derivatives, analogs, isomers, metabolites, salts, solvates, clathrates, polymorphs, and co-crystals as defined above are active, i.e. they exhibit a 5-HT-stimulating or blocking activity. Said activity can be assessed as described above.

It shall further be understood that the 5-HT-stimulating or blocking agents as described herein, or their derivatives, analogs, isomers, metabolites, salts, solvates, clathrates, polymorphs, and co-crystals are preferably in pharmaceutically acceptable or substantially pure form. By pharmaceutically acceptable form, it is meant, inter alia, having a pharmaceutically acceptable level of purity, i.e. excluding normal pharmaceutical additives, such as diluents and carriers, and any material considered toxic at normal dosage levels. In the context of the present invention, purity levels are preferably above 98%, more preferably above 99%, and even more preferably above 99.9%. In a preferred embodiment, said purity level is 99.9%.

For the purpose of the invention, the term "pharmaceutically acceptable" is intended to mean what is useful to the preparation of a pharmaceutical composition, and what is generally safe and non-toxic, for a pharmaceutical use.

A "pharmaceutically acceptable carrier" or "excipient" refers to a non-toxic solid, semi-solid or liquid filler, diluent, encapsulating material or formulation auxiliary of any type. In the pharmaceutical compositions of the present invention, the active principle, alone or in combination with another active principle, can be administered in a unit administration form, as a mixture with conventional pharmaceutical supports, to animals and human beings. Suitable unit administration forms comprise oral-route forms such as tablets, gel capsules, powders, granules and oral suspensions or solutions, sublingual and buccal administration forms, aerosols, implants, subcutaneous, transdermal, topical, intraperitoneal, intramuscular, intravenous, subdermal, transdermal, intrathecal and intranasal administration forms and rectal administration forms. Preferably, the pharmaceutical compositions contain vehicles which are pharmaceutically acceptable for a formulation capable of being injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions. The pharmaceutical forms suitable for injectable use include sterile aqueous solutions or dispersions; formulations including sesame oil, peanut oil or aqueous propylene glycol; and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms, such as bacteria and fungi. Solutions comprising compounds of the invention as free base or pharmacologically acceptable salts can be prepared in water suitably mixed with a surfactant, such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.

### Description of the invention

The present invention relates to the use of antidepressant agents, preferably 5 HT R-stimulating or blocking agents (in particular 5 HT1R-stimulating or blocking agents or 5HT2R-stimulating or blocking agents) to prepare a medicament intended to treat patients that have been diagnosed to be infected with at least one betacoronavirus. In other terms, the invention addresses a 5 HT R-stimulating or blocking agent (in particular 5 HT1R-stimulating or blocking agents or 5HT2R-stimulating or blocking agents) for use for treating viral infections due to at least one betacoronavirus in a patient in need thereof.

The present invention also discloses a method for treating any subject that has been diagnosed to be infected with at least one betacoronavirus, said method comprising administering to said subject an effective amount of a 5 HTR-stimulating or blocking agent (in particular 5 HT1R-stimulating or blocking agents or 5HT2R-stimulating or blocking agents). The method of the invention can comprise a diagnosis step as preliminary step.

It is also likely that antidepressant agents such as 5 HTR-stimulating or blocking agent actually prevent or minimize the infection by a betacoronavirus. This would explain why so few patients hospitalized for said infection in the studied cohort were treated by antidepressants (6,3%), whereas the overall percentage in the French population is higher, estimated at approximatively 10% according to the French health insurance system, based on the proportion of French people who is reimbursed for at least one antidepressant. Antidepressant compounds - and 5 HTR-stimulating or blocking agents in general - may therefore prevent the virus to infect patients and/or lower its symptoms, preventing them to be hospitalized. This effect could be due to the anti-inflammatory and/or to the antiviral effect of the antidepressants.

In a preferred embodiment, the 5 HTR-stimulating or blocking agent of the invention is used for treating patients infected by a Severe Acute Respiratory Syndrome-related coronavirus (SARS-CoV), in particular SARS-CoV-2.

In a preferred embodiment of the invention, the agents of the invention are intended to treat patients suffering from a symptomatic COVID-19 disease, and advantageously from a strong or severe symptomatic COVID-19 disease, as defined above. In a particular embodiment, the treatment of the invention is administered to subjects (animals or humans) that experience for example difficulty in breathing and/or a lack of oxygen.

In another preferred embodiment of the invention, the agents of the invention are capable of preventing and/or treating acute respiratory distress syndrome associated to said viral infection, of lowering the risk of intubation and / or of increasing the survival rate of patients infected by said betacoronavirus, as described in the examples below.

The said infection can be diagnosed or detected by any conventional means, such as molecular biological detection and/or viral titration of respiratory specimens, or by assaying blood for antibodies specific for said betacoronavirus.

In a preferred embodiment, said subject is a human patient. Said patient can be older than 75 years old, between 65 and 74 years old, between 18 and 65 years old, or below 18 years old. In a more preferred embodiment, said human patient is older than 50 years old, even more preferably older than 70 years old. These people indeed usually have greater mortality rates than younger people when infected with said virus.

In a preferred embodiment, said subject is an animal chosen in the group consisting of: bats, hedgehog, camels, mice, humans, pigs, cats, among others. Any other animal that can be infected with a betacoronavirus could also benefit from the treatment of the invention.

The agent of the invention is preferably contained in a pharmaceutical composition. This pharmaceutical composition, herein also called the "pharmaceutical composition of the invention", therefore comprises the 5 HT R-stimulating or blocking agent of the invention and a pharmaceutically acceptable carrier, which can be as defined above.

The pharmaceutical composition of the invention can contain any antidepressant agent that can lower the risk of intubation and/or death of a COVID patient. In particular, said agent can be a 5-HT R-stimulating or blocking agent, including escitalopram, citalopram, sertraline, fluoxetine, paroxetine, mirtazapine, venlafaxine, duloxetine, or clomipramine, as demonstrated by the inventors in the examples below.

More generally, it is possible to use, in the context of the invention, any antidepressant agent and any antimigraine drug, as well as their pharmaceutically acceptable derivatives, analogs, isomers, metabolites, salts, solvates, clathrates, polymorphs, and co-crystals thereof, and combinations thereof, provided that it can lower the risk of intubation and/or death of a COVID patient.

In a particular embodiment, said antidepressant or antimigraine drug is a 5-HT1 BR-stimulating or blocking agent, as defined above.

In a preferred embodiment, the present invention relates to the use of a 5 HT1 BR-stimulating or blocking agent to prepare a medicament intended to treat patients that have been diagnosed to be infected with at least one betacoronavirus. In other terms, the invention addresses a 5 HT1 BR-stimulating or blocking agent for use for treating viral infections due to at least one betacoronavirus in a patient in need thereof.

In a more particular embodiment, the 5-HT1 BR-stimulating or blocking agent according to the invention is an antimigraine drug chosen among triptans and ergotamine. Triptans are well-known in the art as tryptamine-based drugs used in the treatment of migraines and cluster headaches, thanks to their agonistic effects on 5-HT1 BR and 5-HT1 DR. Examples of triptans according to the invention include, but are not limited to, sumatriptan, rizatriptan, zolmitriptan, eletriptan, almotriptan, frovatriptan, naratriptan, avitriptan, and donitriptan. Non limitative examples of salts of said compounds are donitriptan hydrochloride, eletriptan hydrobromide, and rizatriptan benzoate.

The 5-HT1 BR-stimulating or blocking agents according to the invention can alternatively be selected among antidepressant agents. By "antidepressant agent", it is meant herein an active agent that is capable to treat mood disorders, such as depression (including severe depression) and/or dysthymia, and/or anxiety disorders. Antidepressant agents according to the invention include, without limitation, serotonin reuptake inhibitors (SRIs); tricyclic antidepressants (TCAs); monoamine oxidase inhibitors (MAOs).

Serotonin reuptake inhibitors (SRIs) designate a class of compounds that typically act by inhibiting the reuptake of the serotonin neurotransmitter into the presynaptic terminal, thereby increasing the serotonin extracellular level and thus serotoninergic transmission. Such compounds can act selectively or non-selectively on the neurotransmitter serotonin. SRIs can indeed also display various degrees of selectivity towards the other monoamine reuptake systems, in particular the transporters for norepinephrine and dopamine.

SRIs typically include selective serotonin reuptake inhibitors (SSRIs), serotonine and norepinephrine reuptake inhibitors (SNRIs) and serotonin-norepinephrine-dopamine reuptake inhibitor (SNDRIs).

Examples of selective serotonin reuptake inhibitors (SSRIs) that can be used in the invention include, without limitation, fluoxetine, citalopram, escitalopram, sertraline, norsertraline, paroxetine, fluvoxamine, vortioxetine, femoxetine, indalpine, alaproclate, cericlamine, ifoxetine, zimelidine, dapoxetine, and etoperidone. In a preferred embodiment, they are chosen among citalopram, escitalopram, sertraline, norsertraline, paroxetine, fluvoxamine, vortioxetine, femoxetine, indalpine, alaproclate, cericlamine, ifoxetine and zimelidine. Fluoxetine is preferably not encompassed in the list of the SSRIs useful in the invention. In other words, the agent of the invention is preferably a SSRI provided that it is not Fluoxetine.

Examples of active SSRIs metabolites that can be used in the invention include, without limitation, desmethylcitalopram, didesmethylcitalopram, and seproxetine (i.e. (S)-norfluoxetine).

Examples of serotonine and norepinephrine reuptake inhibitors (SNRIs) that can be used in the invention include, without limitation, duloxetine, venlafaxine, desvenlafaxine, milnacipran, levominalcipran and sibutramine.

Examples of serotonin-norepinephrine-dopamine reuptake inhibitor (SNDRIs) (also known as triple reuptake inhibitor or TRI) that can be used in the invention include, without limitation, bicifadine, brasofensine, tesofensine and nomifensine.

Examples of tricyclic antidepressants (TCAs) that can be used in the invention include, without limitation, clomipramine, amoxapine, nortriptyline, maprotiline, trimipramine, imipramine, desipramine and protriptyline.

Examples of monoamine oxidase inhibitors (MAOs) that can be used in the invention include, without limitation, iproniazide, phenelzine, tranylcipromine, moclobemide, selegiline and rasagiline.

Other suitable 5-HT1 BR-stimulating agents that can be used in the invention are for example: anpirtoline hydrochloride, CGS-12066A, CGS 12066B dimaleate, oxymetazoline, 5-carboxamidotryptamine, CP-93129 and salts thereof (such as CP-93129 dihydrochloride), CP-94253 and salts thereof (such as CP-94253 hydrochloride), CP-122,288, CP-135,807, RU-24969 and salts thereof (such as RU-24969 hemisuccinate), ziprasidone, asenapine, 5-nonyloxytryptamine oxalate, pindolol and (S)-(-)-pindolol and the like.

The 5 HT R- blocking agent of the invention can act by blocking presynaptic alpha-2 adrenergic receptors. Such blockers include, among others, mirtazapine, aptazapine, esmirtazapine, setiptiline and S32212 (also known as N-[4-methoxy-3-(4-methylpiperazin-1-yl)phenyl]-1,2-dihydro-3H-benzo[e]indole-3-carboxamide).

The 5 HT R-stimulating or blocking agent of the invention can also be an atypical antidepressant (defined as such as they do not belong to any of the foregoing classes of antidepressants), for example, without limitation, tianeptine, agomelatine, nefazodone, trazodone, buspirone, tandospirone, and ketamine.

The 5 HT R-stimulating or blocking agent of the invention can also be a 5 HT2 R stimulating or blocking agent, such as agomelatine or Saint John's wort.

Any of these antidepressant compound can be used in the composition of the invention.

In a more preferred embodiment, the 5-HTR-stimulating or blocking agent used in the composition of the invention is chosen in the group consisting of: escitalopram, venlafaxine, citalopram, sertraline, fluoxetine, paroxetine, duloxetine, mirtazapine, or clomipramine. In an even more preferred embodiment, the 5-HTR-stimulating or blocking agent used in the composition of the invention is chosen in the group consisting of: escitalopram, venlafaxine, citalopram, sertraline, paroxetine, duloxetine, mirtazapine or clomipramine.

Of note, tetracyclic antidepressants (such as mianserine) are not encompassed within the scope of the invention. As a matter of fact, these compounds do not act on the 5 HT receptor. The inventors have found that patients treated with such antidepressants displayed the same symptoms and outcome as compared with non-treated patients. This observation strengthens the hypothesis that the 5 HT receptors are involved in the effect of antidepressants on disease severity, possibly through reduction of inflammation and/or virus activity.

The composition of the invention can contain a combination of at least two antidepressant agents as defined above.

The daily dose of the agent(s) to be administered to the infected subject is preferably the conventional dose used in the art for antidepressant purposes. This dose is for example comprised between about 5 mg/day and about 60 mg/day for fluoxetine-equivalent dose, said dose being as defined in²⁷. Preferably, low to moderate doses of 10 mg/day to 30 mg/day are used in the context of the invention among adults aged more than 70 years old of age, so as to minimize side effects. As a matter of fact, low and moderate doses of antidepressants are generally well tolerated, especially when they are used on a short period, including in older adults who are the most prone to develop severe Covid-19 infection.¹²⁻¹⁴

According to a preferred embodiment, said 5-HTR-stimulating or blocking agent can be administered to the subject at an effective Fluoxetine-equivalent dose comprised between about 5 mg/day and about 60 mg/day, preferably between about 10 mg/day and about 30 mg/ day, more preferably between about 10 mg/day and about 20 mg/day for parenteral or oral administration.

In other words, the dose of antidepressant contained in the composition of the invention is such that, when converted into dose-equivalent to fluoxetine-equivalent dose as explained in Hayasaka Y²⁷, it is comprised in the above-mentioned ranges.

According to this study, the following doses of other antidepressant agents are equivalent to 40mg/day of Fluoxetine:
- paroxetine : 34.0mg/day,
- bupropion : 48.5mg/day,
- clomipramine : 116.1mg/day,
- desipramine : 196.3mg/day,
- escitalopram : 18.0mg/day,
- fluvoxamine : 143.3mg/day,
- mirtazapine 50.9mg/day,
- sertraline 98.5mg/day,
- venlafaxine 149.4mg/day.

These doses are thus recommended as highest dose in the treatment of the invention.

Yet, according to another preferred embodiment, said 5-HTR-stimulating or blocking agent can be administered to the subject at an effective Fluoxetine-equivalent dose orally or by intravenous perfusion comprised between 5 mg and 60 mg per day, and preferably between 5 mg and 30 mg in older adults aged 70 years and over, and between 20 mg and 60 mg in younger adults aged 18 to 69 years old of age.

It is within the skill of the person in the art to determine the desired therapeutic amount of 5-HTR-stimulating or blocking agent to deliver by routine methods in the art.

The pharmaceutical composition of the invention may preferably be in a form suitable for the purposes of the invention. For example, said composition may be in a form suitable for parenteral or oral administration, such as a liquid suspension, or a solid dosage form (granules, pills, capsules or tablets). The term "parenteral" as used herein includes subcutaneous injection, intravenous, or intramuscular, injection.

In a preferred embodiment, the agent of the invention is administered to the patient once it is diagnosed from severe COVID disease, and until the patient displays at least one symptom of the COVID disease. The treatment preferably ceases when the infection is over. Such short-term treatments are well tolerated and do not induce any side-effect or dependency in the treated patients.

### FIGURE LEGENDS

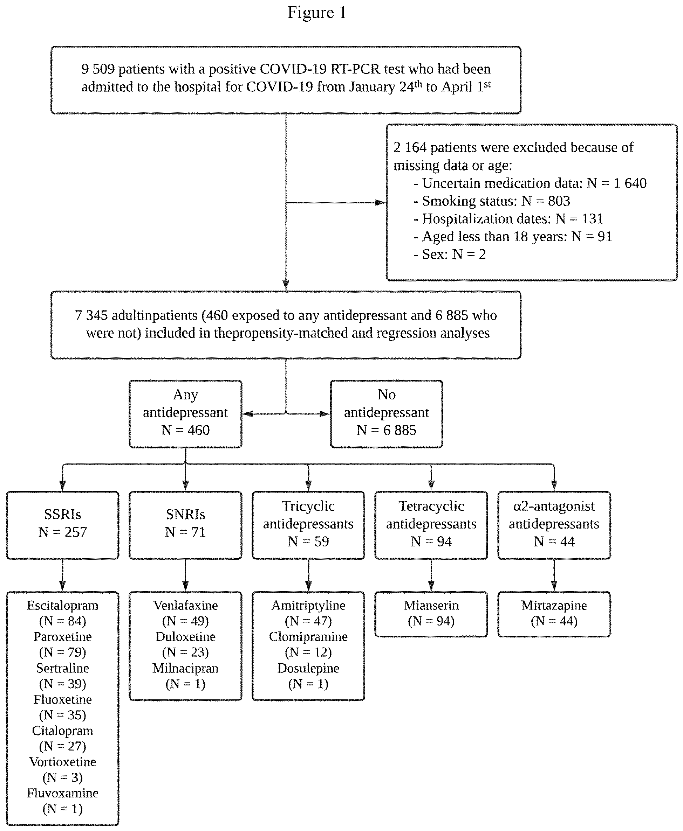

**Figure 1** describes the characteristics of the studied cohort.

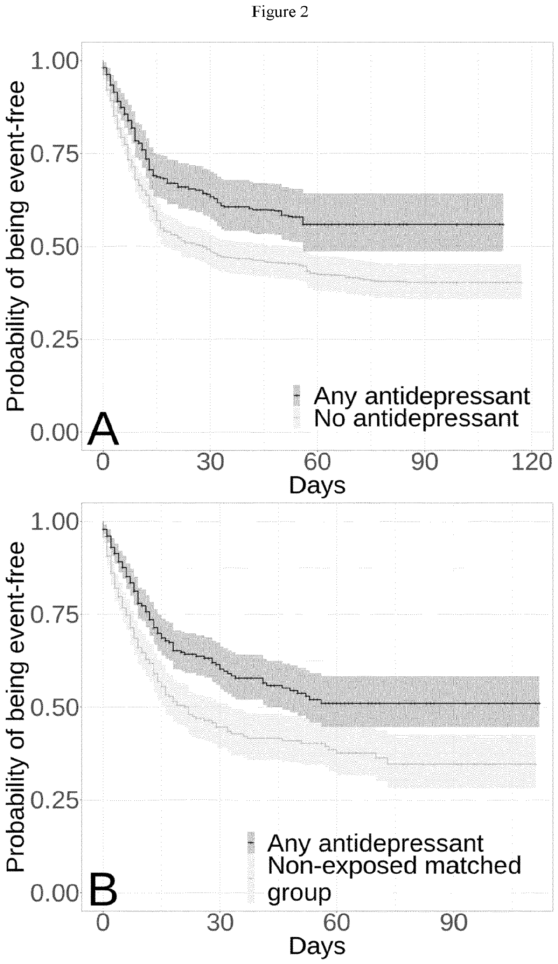

**Figure 2** shows the freedom from the composite endpoint of intubation or death in the full sample (N=7,345) (A) and in the matched analytic sample (N=920) (B) of patients who had been admitted to the hospital with Covid-19 according to antidepressant exposure. *Note:* The shaded areas represent pointwise 95% confidence intervals.

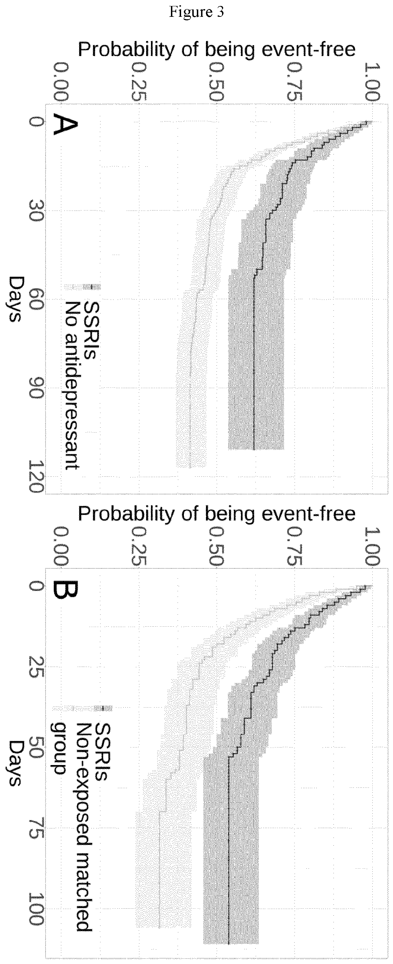

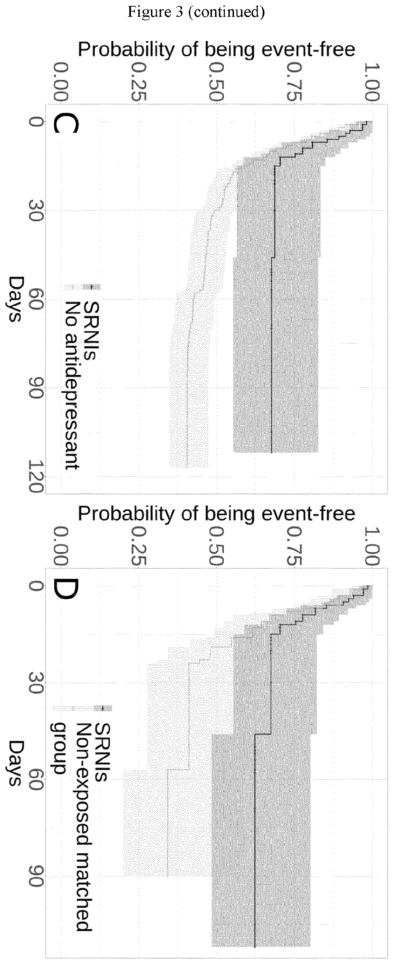

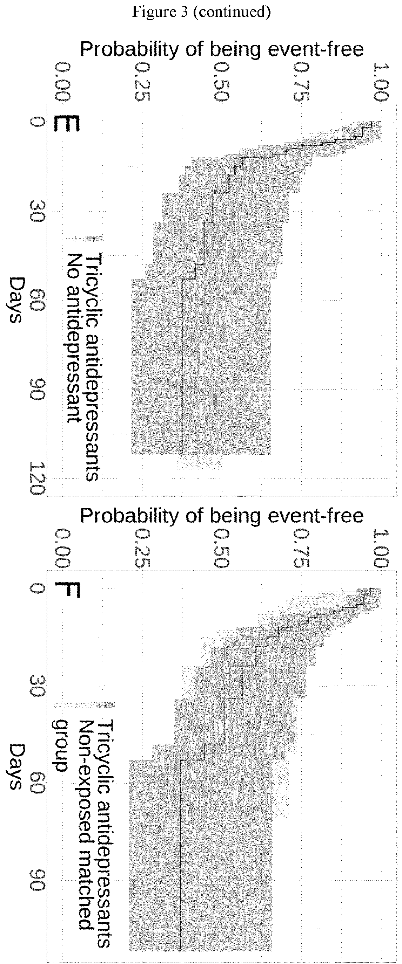

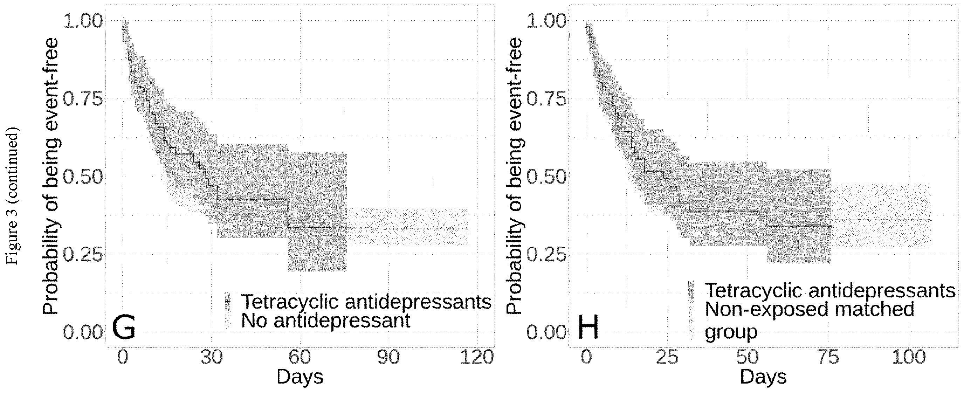

**Figure 3** discloses the freedom from the composite endpoint of intubation or death in the full sample (A, C, E, G, and I) and in the matched analytic sample (B, D, F, H, and J) of patients who had been admitted to the hospital with Covid-19 according to exposure to classes of antidepressants: SSRIs (A, B), SNRIs (C, D), tricyclic antidepressant (E, F), tetracyclic antidepressants (G, H). *Note:* The shaded areas represent pointwise 95% confidence intervals.

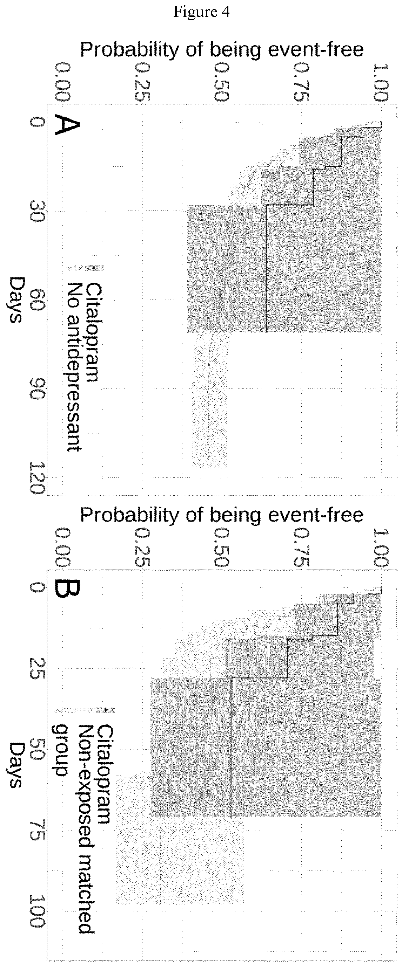

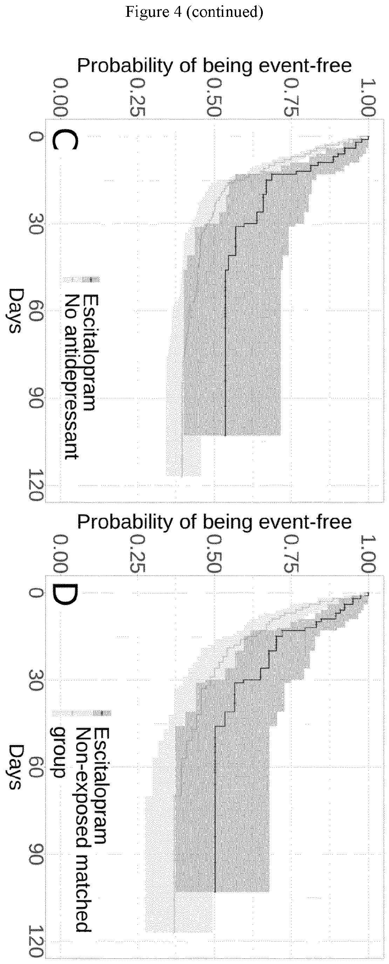

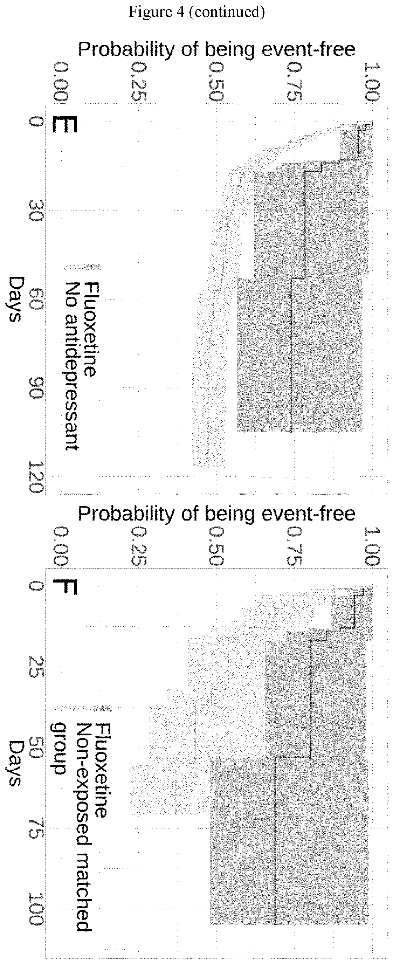

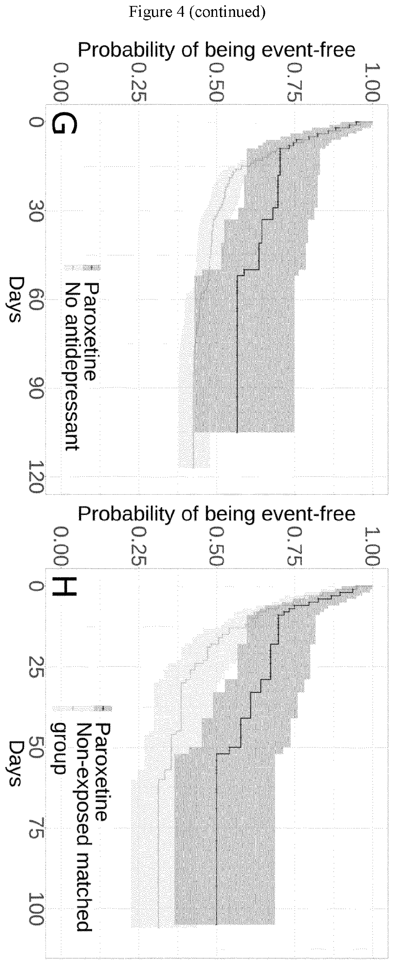

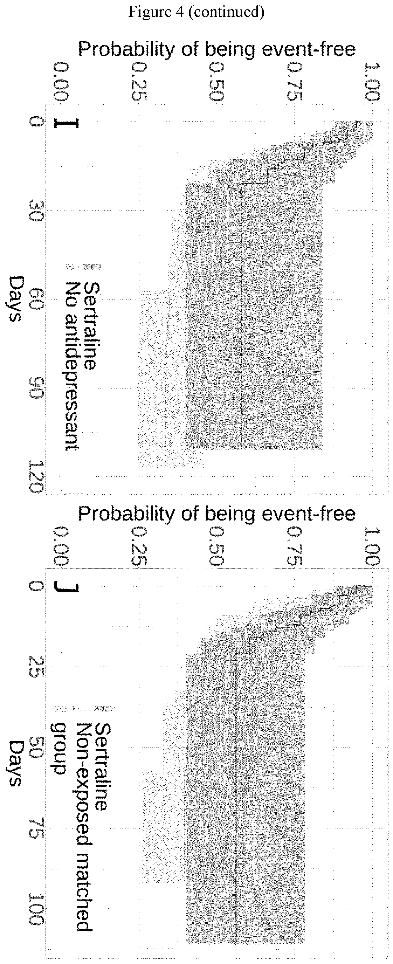

**Figure 4** discloses the freedom from the composite endpoint of intubation or death in the full sample (A, C, E, G, and I) and in the matched analytic sample (B, D, F, H, and J) of patients who had been admitted to the hospital with Covid-19 according to exposure to individual SSRI medications: citalopram (A, B), escitalopram (C,D), Fluoxetine (E,F), paroxetine (G,H), sertraline (I,J). Note: The shaded areas represent pointwise 95% confidence intervals.

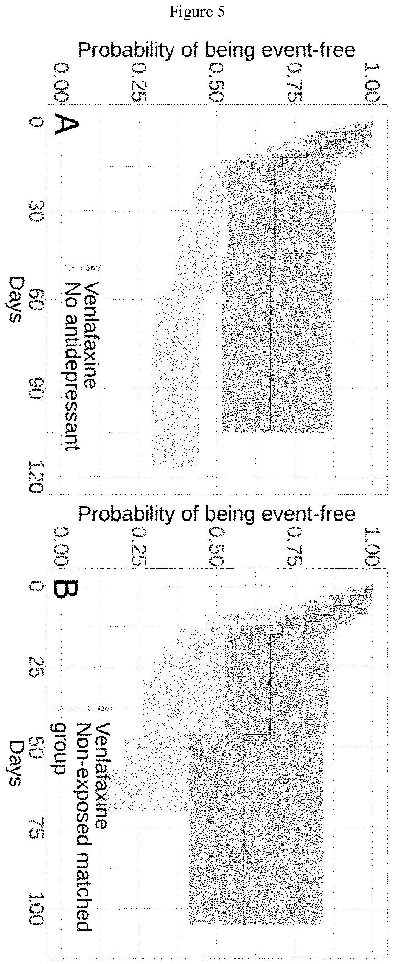

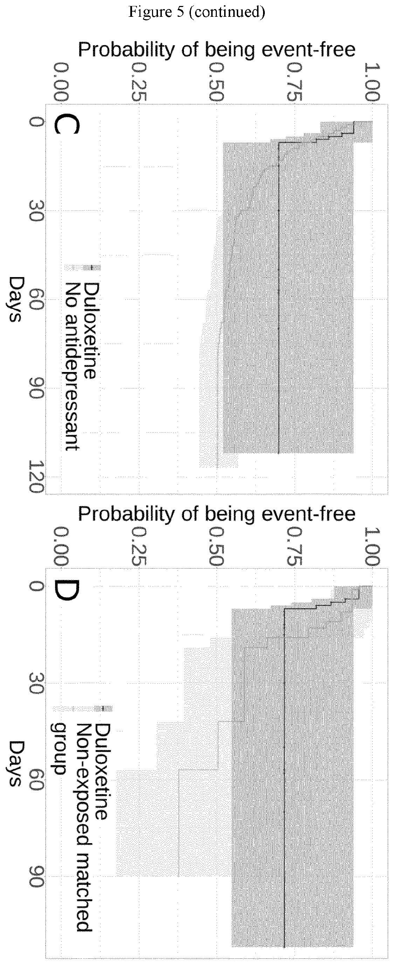

**Figure 5** shows the freedom from the composite endpoint of intubation or death in the full sample (A, C) and in the matched analytic sample (B, D) of patients who had been admitted to the hospital with Covid-19 according to exposure to individual SNRI medications: venlafaxine (A,B), duloxetine (C,D). Note: The shaded areas represent pointwise 95% confidence intervals.

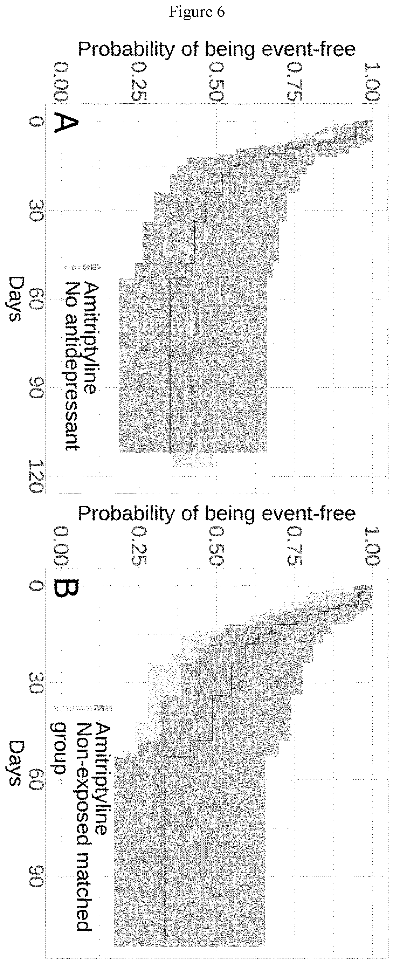

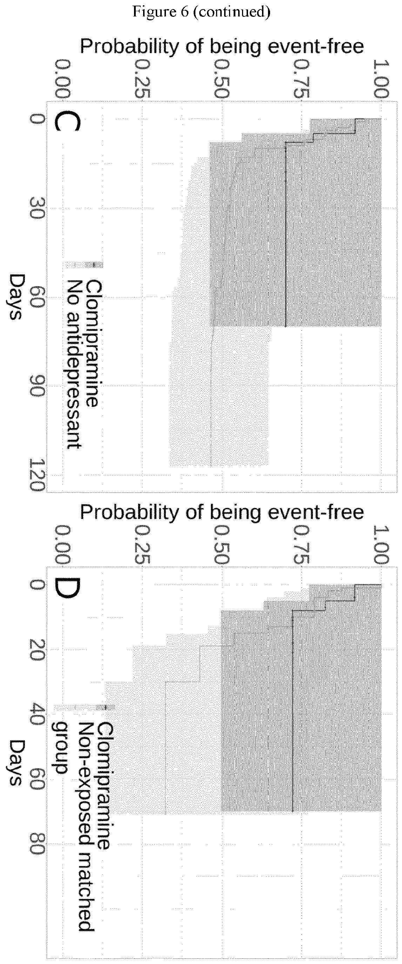

**Figure 6** shows the freedom from the composite endpoint of intubation or death in the full sample (A, C) and in the matched analytic sample (B, D) of patients who had been admitted to the hospital with Covid-19 according to exposure to individual tricyclic antidepressant medications: amitriptyline (A,B) or clomipramine (C,D). *Note:* The shaded areas represent pointwise 95% confidence intervals.

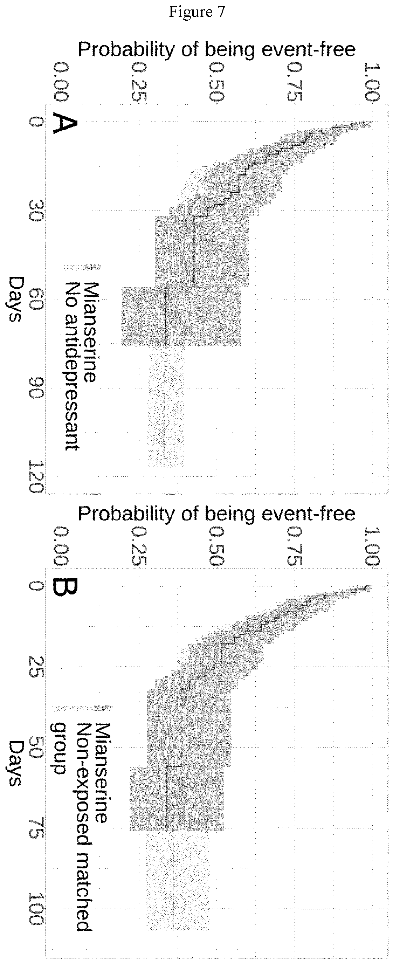

**Figure 7** shows the freedom from the composite endpoint of intubation or death in the full sample (A) and in the matched analytic sample (B) of patients who had been admitted to the hospital with Covid-19 according to mianserine exposure (tetracyclic antidepressant). *Note:* The shaded areas represent pointwise 95% confidence intervals.

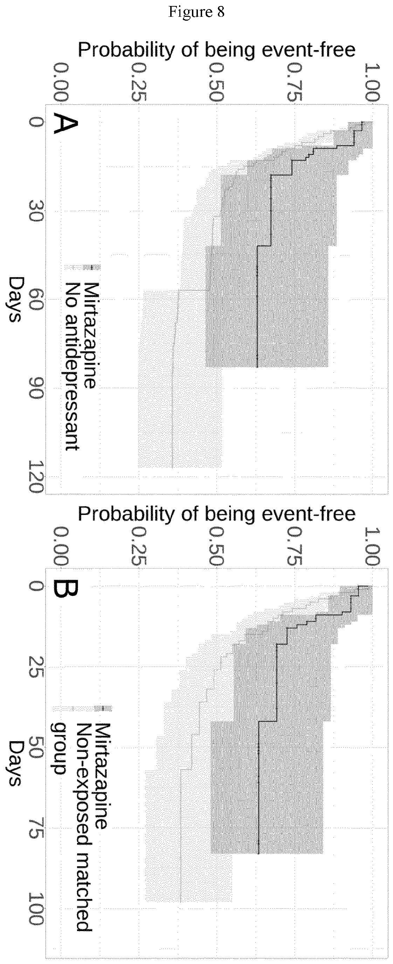

**Figure 8** shows the freedom from the composite endpoint of intubation or death in the full sample (A) and in the matched analytic sample (B) of patients who had been admitted to the hospital with Covid-19 according to mirtazapine exposure (α2-antagonist antidepressant). *Note:* The shaded areas represent pointwise 95% confidence intervals.

### EXAMPLES

Although the present invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the spirit and scope of the present invention as defined by the appended claims.

### 1. MATERIAL AND METHODS

### 1.1. Setting

We conducted this study at AP-HP, which comprises 39 hospitals, 23 of which are acute, 20 adult and 3 pediatric hospitals. We included all adults aged 18 years or over who have been admitted to the hospital from January 24st until May 20th with Covid-19, ascertained by a positive reverse-transcriptase-polymerase-chain-reaction (RT-PCR) test from analysis of nasopharyngeal or oropharyngeal swab specimens. The Institutional Review Board of the AP-HP clinical data warehouse approved this study on April 8th (CSE-20-20_COVID19). All procedures related to this work adhered to the ethical standards of the relevant national and institutional committees on human experimentation and with the Helsinki Declaration of 1975, as revised in 2008.

### 1.2. Data sources

We used data from the AP-HP Health Data Warehouse ('Entrepôt de Données de Santé (EDS)'). This warehouse contains all the clinical data available on all inpatient visits for Covid-19 to any of the 39 Greater Paris University hospitals. The data obtained included patients' demographic characteristics, vital signs, laboratory test and RT-PCR test results, medication administration data, past and current medication lists, past and current diagnoses, discharge disposition, ventilator use data, and death certificates.

### 1.3. Variables assessed

We obtained the following data for each patient at the time of the hospitalization: sex; age, which was categorized based on the OpenSAFELY results¹⁵ (i.e. 18-50, 51-70, 71-80, 81+); obesity (defined as having a body-mass index higher than 30 kg/m2 or an International Statistical Classification of Diseases and Related Health Problems (ICD-10) diagnosis code for obesity (E66.0, E66.1, E66.2, E66.8, E66.9); self-reported smoking status; any medical condition associated with increased risk of severe Coved-19 infection,¹⁵⁻¹⁸ based on ICD-10 diagnosis codes, including diabetes mellitus (E11), diseases of the circulatory system (100-199), diseases of the respiratory system (J00-J99), neoplasms (C00-D49), and diseases of the blood and blood-forming organs and certain disorders involving the immune mechanism (D5-D8); any medication prescribed according to compassionate use or as part of clinical trials (e.g., hydroxychloroquine, azithromycin, remdesivir, tocilizumab, or sarilumab); clinical severity of Covid-19 at admission, defined as having at least one of the following criteria:¹⁹ respiratory rate > 24 breaths/min or < 12 breaths/min, resting peripheral capillary oxygen saturation in ambient air < 90% , temperature > 40°C, or systolic blood pressure < 100 mm Hg; and biological severity of Covid-19 at admission, defined as having at least one of the following criteria:^{19,20} increased neutrophil-to-lymphocyte ratio or low lymphocyte-to-C-reactive protein ratio (both variables were dichotomized by the median values in the full sample), or plasma lactate levels higher than 2 mmol/L. To take into account possible confounding by indication bias for antidepressants, we recorded whether patients had any current mood or anxiety disorder (F30-F48) or any other current psychiatric disorder (F00-F29 and F50-F99), and whether they were prescribed any benzodiazepine or Z-drug, any mood stabilizer medication (i.e., lithium or antiepileptic medications with mood stabilizing effects), or any antipsychotic medication.

All medical notes and prescriptions are computerized in Greater Paris University hospitals. Medications and their mode of administration (i.e., dosage, frequency, date, condition of intake) were identified from medication administration data or scanned hand-written medical prescriptions, through two deep learning models based on BERT contextual embeddings,²¹ one for the medications and one for their mode of administration. The model was trained on the APmed corpus,²² a previously annotated dataset for this task. Extracted medications names were then normalized to the Anatomical Therapeutic Chemical (ATC) terminology using approximate string matching.

### 1.4. Exposure to antidepressants

Study baseline was defined as the date of hospital admission. Patients were considered to have been exposed to an antidepressant if they received it at any time during the follow-up period, from study baseline to the end of the hospitalization or intubation or death.

### 1.5. Endpoints

The primary endpoint was the time from study baseline to intubation or death. For patients who died after intubation, the timing of the primary endpoint was defined as the time of intubation.

### 1.6. Statistical analysis

We calculated frequencies and means (± standard deviations (SD)) of each variable described above in patients exposed or not to antidepressants and compared them using chi-square tests or Welch's t-tests. Patients without an end-point event had their data censored on May 20th, 2020.

To examine the association of any antidepressant, classes of antidepressants, and individual treatments with the primary composite endpoint of intubation or death, we performed Cox proportional-hazards regression models. Weighted Cox regression models were used when proportional hazards assumption was not met. To help account for the nonrandomized prescription of antidepressants and reduce the effects of confounding, the primary analysis used propensity score analysis with inverse probability weighting.^{23,24} The individual propensities for exposure were estimated by multivariable logistic regression models that included sex, age, obesity, self-reported smoking status, any medical condition, any medication prescribed according to compassionate use or as part of clinical trials, clinical and biological severity of Covid-19 at admission, any current mood or anxiety disorder, any other current psychiatric disorder, any prescribed benzodiazepine or Z-drug, mood stabilizer, or antipsychotic medication. In the inverse-probability-weighted analyses, the predicted probabilities from the propensity-score models were used to calculate the stabilized inverse-probability-weighting weights.²³ Association between any antidepressant, classes of antidepressants, and individual treatments and the primary endpoint was then estimated using multivariable Cox regression models. Kaplan-Meier curves were performed using the inverse-probability-weighting weights,²⁵ and their pointwise 95% confidence intervals were estimated using the nonparametric bootstrap method.²⁶

We conducted sensitivity analyses, including multivariable Cox regression models comprising as covariates the same variables as the inverse-probability-weighted analyses, and univariate Cox regression models in matched analytic samples. For these latter analyses, we selected one control for each exposed case for any antidepressant and each classe of antidepressants, and two controls for each exposed case for individual antidepressant medications, based on the same variables used for both the inverse-probability-weighted and the multivariable Cox regression analyses. To reduce the effects of confounding, optimal matching was used in order to obtain the smallest average absolute distance across all clinical characteristics between exposed patient and non-exposed matched controls. We also performed multivariable Cox regression models including interaction terms to examine whether the association between exposure to any antidepressant and the outcome differ by subgroups defined by baseline characteristics.

Within the group of patients exposed to antidepressants, we tested the association of daily dose (converted into dose-equivalent to fluoxetine-equivalent dose²⁷ and dichotomized by the median value among patients taking antidepressants) with the primary endpoint. Furthermore, we examined whether exposure to a combination of antidepressants significantly modified the risk of death or intubation compared to being exposed to only one antidepressant treatment.

Finally, we reproduced these analyses (i) among patients with Covid-19 who took antidepressants before hospital admission, ascertained by an ongoing medical prescription dated of less than 3 months before being admitted, but not during the hospitalization, (ii) among patients with critical infections in intensive care units (ICUs), and (iii) using death and intubation as separate endpoints.

For all significant associations, we performed residual analyses to assess the fit of the data, check assumptions, including proportional hazards assumptions, and examined the potential influence of outliers. To improve the quality of result reporting, we followed the recommendations of The Strengthening the Reporting of Observational Studies in Epidemiology (STROBE) Initiative.²⁸ Statistical significance was fixed a priori at p<0.05. All analyses were conducted between May 25th and June 17th in R software version 2.4.3.

### II. RESULTS

### 2.1. Characteristics of the cohort

Of the 9,509 patients with positive Covid-19 RT-PCR test who had been admitted to the hospital, a total of 2,164 patients (22.8%) were excluded because of missing data or their young age (i.e. less than 18 years old of age). Of the remaining 7,345 adult inpatients, 460 patients (6.3%) received antidepressants during the hospitalization, at a mean fluoxetine-equivalent dose of 16.9 mg (SE=0.7) per day. Of them, 65 patients (14.1%) were exposed to antidepressants from two different classes. The mean dosage in patients exposed to any antidepressant was 16.9 (SE=0.7) fluoxetine-equivalent milligrams. Doses of each antidepressant medication are detailed in **Table 3.**

RT-PCR test results were obtained after a median delay of 1 day (SD=12.1) from hospital admission date. This delay was not significantly different between exposed and non-exposed patients [median in the antidepressant group=1 day (SD=14.1); median in the non-exposed group=1 day (SD=11.9); Mood's median test Chi-square=0.21, p=0.650)].

Over a mean follow-up of 18.6 days (SE=0.3; median=4 days; range: 1 day to 117 days), 1,331 patients (18.1%) had a primary end-point event at the time of data cutoff on May 20^{th}. In patients exposed to antidepressants, the mean follow-up was 20.1 days (SE=1.1; median=11 days; range: 1 day to 112 days), while it was of 18.4 days (SE=0.3; median=4 days; range: 1 day to 117 days) in those who were not. The times to follow-up by medication exposure are given in **Table 4.**

All baseline characteristics were independently and significantly associated with the primary endpoint, except for smoking status, any medication prescribed according to compassionate use or as part of a clinical trial, and any current mood or anxiety or other psychiatric disorder **(Table 1)**.

The distributions of the patients' characteristics according to antidepressant exposure, and exposure by classes of antidepressants and individual treatments are shown in **Tables 5 to 15.**

In the full sample, antidepressant exposure significantly differed according to all characteristics, as did exposure to SSRIs (except for any medication prescribed according to compassionate use or as part of a clinical trial), SNRIs (except for sex and any medication prescribed according to compassionate use or as part of a clinical trial), tricyclic antidepressants (except for smoking status and any current psychiatric disorder outside mood and anxiety disorders), tetracyclic antidepressant (except for sex, obesity, smoking status, and any medication prescribed according to compassionate use or as part of a clinical trial), and α2-antagonist antidepressants (except for obesity, smoking status, any medication prescribed according to compassionate use or as part of a clinical trial, any current psychiatric disorder outside mood and anxiety disorders, and clinical severity of Covid-19), and the direction of associations indicated older age and greater medical severity of exposed people than those who were not. After applying the propensity score weights, these differences were substantially reduced but remained significant for several variables. In the matched analytic samples, there were no significant differences in any characteristic across the different exposures, except for antidepressant exposure, for which sex significantly differed **(Tables 5 to 15).**

Among patients receiving antidepressants, SSRIs, SNRIs, tricyclic antidepressants, tetracyclic antidepressants, and α2-antagonist antidepressants, the primary endpoint of death or intubation occurred in 143 patients (31.1%), 75 patients (29.2%), 18 patients (25.4%), 19 patients (32.2%), 43 patients (45.7%) and 12 patients (27.3%), respectively, whereas 1,188 (17.3%) non-exposed patients had this outcome **(Table 2).** When adjusting for the older age and the greater medical severity of patients receiving antidepressants, the primary multivariable analysis with inverse probability weighting according to the propensity score showed a significant negative association of the composite endpoint with exposure to any antidepressant (HR, 0.64; 95% CI, 0.51 to 0.80, p<0.001) **(****Figure 2****)**, SSRIs (HR, 0.56; 95% CI, 0.42 to 0.75, p<0.001), and SNRIs (HR, 0.57; 95% CI, 0.34 to 0.96, p=0.034) **(****Figure 3****)**, Among hospitalized patients with Covid-19, exposure to escitalopram (HR, 0.61; 95% CI, 0.40 to 0.92, p=0.018), fluoxetine (HR, 0.32; 95% CI, 0.14 to 0.73, p=0.007), or venlafaxine (HR, 0.47; 95% CI, 0.24 to 0.91, p=0.025) was significantly associated with a reduced risk of intubation or death. There were no significant effects of baseline characteristics on the association between exposure to any antidepressant and the outcome, except for biological severity of Covid-19 at admission **(Table 16).**

In sensitivity analyses, multivariable Cox regression models in the full sample yielded similar results, as did univariate Cox regression models in the matched analytic samples, except exposures to paroxetine (HR, 0.62; 95% CI, 0.40 to 0.96, p=0.034) and mirtazapine (HR, 0.52; 95% CI, 0.27 to 0.99, p=0.046), which were significantly associated with a reduced risk of death or intubation in these analyses **(Table 2;** **Figures 4 to 8****).**

Exposure to higher rather than lower doses of antidepressants was not significantly associated with the composite outcome, except for tetracyclic antidepressants, for which higher doses were associated with increased risk of death or intubation **(Table 17).** Similarly, exposure to a combination of antidepressants did not significantly modify this risk compared to being exposed to only one antidepressant (not shown).

When using death and intubation as separate endpoints, we found that exposure to any antidepressant and SSRIs significantly reduced both risks across all multivariable analyses **(Table 18**).

Among patients admitted to ICUs, exposure to any antidepressant was significantly associated with a reduced risk of death or intubation in the primary multivariable Cox model with inverse probability weighting, but not in other multivariable analyses, probably because of lack of statistical power, as only 28 patients (4.7%) in ICUs received an antidepressant **(Table 19).**

Finally, among the 159 patients with Covid-19 who had an ongoing antidepressant treatment stopped at the time of hospital admission, we found no significant association between exposure to antidepressants or SSRIs prior to hospital admission and the primary endpoint **(Table 20**).

### III. DISCUSSION

In this observational multicenter study involving a large number of patients admitted to the hospital with Covid-19, the risk of intubation or death was significantly and substantially reduced in patients who received SSRIs or SNRIs, and specifically fluoxetine, escitalopram and venlafaxine, than in those who did not. This association was observed with a mean dosage of 16.9 (SE=0.7) fluoxetine-equivalent milligrams.

This is, to our knowledge, the first study examining the association of the use of these medications with risk of death or intubation in a clinical population of patients with Covid-19. The present results suggest that SSRIs and SNRIs, and specifically fluoxetine, escitalopram and venlafaxine, can significantly and substantially reduce the risk of intubation or death in patients admitted to the hospital with Covid-19.

In the analyses, we tried to minimize the effects of confounding in several different ways. First, we used multivariable regression models with inverse probability weighting to minimize the effects of confounding by indication.^{23,24} We also performed sensitivity analyses, including multivariable Cox regression models and univariate Cox regression models in matched analytic samples, that showed similar results, giving strength to our conclusion. Second, although some amount of unmeasured confounding may remain, our analyses adjusted for numerous potential confounders, including sex, age, obesity, self-reported smoking status, any medical condition, any medication prescribed according to compassionate use or as part of clinical trials, clinical and biological severity of Covid-19 at admission, any current mood or anxiety disorder, any other current psychiatric disorder, any prescribed benzodiazepine or Z-drug, mood stabilizer, or antipsychotic medication. Furthermore, our findings indicate that these associations were not significantly different across subgroups defined by baseline characteristics, except for biological severity of Covid-19 at hospital admission, and only observed for patients receiving these medications during the visit, but not if they were stopped these treatments at the date of hospital admission. Third, these associations remained significant when using death and intubation as separate endpoints. Finally, the lower rate of exposure to antidepressants among patients who were admitted to ICUs (4.7%) than in those who were not (6.4%), and the significant association of this exposure with reduced risk of death or intubation in this population in the primary analysis only, reinforce our conclusion.

In this observational study involving patients with Covid-19 who had been admitted to the hospital, the risk of death or intubation was significantly and substantially reduced among those who received SSRIs or SNRIs, and specifically fluoxetine, escitalopram and venlafaxine at low to moderate dosages than among those who did not. Double-blind controlled randomized clinical trials of these specific medications are urgently needed.

**Table 1. Associations of baseline clinical characteristics with the composite endpoint of intubation or death in the cohort of adult inpatients with Covid-19 (N=7,345).**

| | | | | Composite endpoint of intubation or death | | | | |
|---|---|---|---|---|---|---|---|---|
| | Full population N = 7,345 | With outcome N = 1,331 | Without the outcome N = 6,014 | Crude analysis | | Mutivariable analysis | | |
| | N (%) | N (%) | N (%) | HR (SE) | p-value | HR (SE) | p-value | Collinearity diagnosis (VIF) |
| *Characteristics* Age (years) | | | | | | | | 1.06 |
| *18-50* | 2709 (36.9%) | 131 (9.84%) | 2578 (42.9%) | Ref. | | Ref. | | |
| *51-70* | 2530 (34.4%) | 436 (32.8%) | 2094 (34.8%) | 3.24 (0.10) | <0.001* | 1.81 (0.10) | <0.001* | |
| *71-80* | 942 (12.8%) | 323 (24.3%) | 619 (10.3%) | 5.61 (0.10) | <0.001* | 2.77 (0.11) | <0.001* | |
| *81+* | 1164 (15.8%) | 441 (33.1%) | 723 (12.0%) | 5.15 (0.10) | <0.001* | 3.25 (0.10) | <0.001* | |
| Sex | | | | | | | | 1.06 |
| *Women* | 3619 (49.3%) | 440 (33.1%) | 3179 (52.9%) | Ref. | | Ref. | | |
| *Men* | 3726 (50.7%) | 891 (66.9%) | 2835 (47.1%) | 2.01 (0.06) | <0.001* | 1.52 (0.06) | <0.001* | |
| Obesity ^{α} | | | | | | | | 1.03 |
| *Yes* | 975 (13.3%) | 314 (23.6%) | 661 (11.0%) | 1.81 (0.06) | <0.001* | 1.55 (0.07) | <0.001* | |
| *No* | 6370 (86.7%) | 1017 (76.4%) | 5353 (89.0%) | Ref. | | Ref. | | |
| Smoking | | | | | | | | 1.03 |
| *Yes* | 623 (8.5%) | 190 (14.3%) | 433 (7.20%) | 1.46 (0.08) | <0.001* | 0.94 (0.08) | 0.413 | |
| *No* | 6722 (91.5%) | 1141 (85.7%) | 5581 (92.8%) | Ref. | | Ref. | | |
| Any medical condition ^{β} | | | | | | | | 1.27 |
| *Yes* | 2574 (35.0%) | 906 (68.1%) | 1668 (27.7%) | 4.36 (0.06) | <0.001* | 2.65 (0.07) | <0.001* | |
| *No* | 4771 (65.0%) | 425 (31.9%) | 4346 (72.3%) | Ref. | | Ref. | | |
| Medication according to compassionate use or as part of a clinical trial ^{θ} | | | | | | | | 1.10 |
| *Yes* | 1240 (16.9%) | 371 (27.9%) | 869 (14.4%) | 1.71 (0.06) | <0.001* | 0.96 (0.07) | 0.619 | |
| *No* | 6105 (83.1%) | 960 (72.1%) | 5145 (85.6%) | Ref. | | Ref. | | |
| Any current mood or anxiety disorder ^{£} | | | | | | | | 1.04 |
| *Yes* | 125 (1.7%) | 52 (3.9%) | 73 (1.2%) | 1.95 (0.14) | 0.026* | 0.74 (0.11) | 0.185 | |
| *No* | 7220 (98.3%) | 1279 (96.1%) | 5941 (98.8%) | Ref. | | Ref. | | |
| Any other current psychiatric disorder 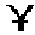 | | | | | | | | 1.08 |
| *Yes* | 304 (4.1%) | 155 (11.6%) | 149 (2.5%) | 2.81 (0.09) | <0.001* | 1.15 (0.09) | 0.142 | |
| *No* | 7041 (95.9%) | 1176 (88.4%) | 5865 (97.5%) | Ref. | | Ref. | | |
| Any benzodiazepine or Z-drug | | | | | | | | 1.11 |
| *Yes* | 752 (10.2%) | 355 (26.7%) | 397 (6.6%) | 2.68 (0.06) | <0.001* | 1.58 (0.07) | <0.001* | |
| *No* | 6593 (89.8%) | 976 (73.3%) | 5617 (93.4%) | Ref. | | Ref. | | |
| Any mood stabilizer medication ^{Ω} | | | | | | | | 1.04 |
| *Yes* | 287 (3.91%) | 84 (6.31%) | 203 (3.4%) | 1.29 (0.11) | <0.001* | 0.74 (0.11) | 0.025* | |
| *No* | 7058 (96.1%) | 1247 (93.7%) | 5811 (96.6%) | Ref. | | Ref. | | |
| Any antipsychotic medication | | | | | | | | 1.09 |
| *Yes* | 264 (3.6%) | 109 (8.19%) | 155 (2.6%) | 1.97 (0.10) | <0.001* | 1.13 (0.11) | 0.252 | |
| *No* | 7081 (96.4%) | 1222 (91.8%) | 5859 (97.4%) | Ref. | | Ref. | | |
| Clinical severity of Covid-19 at admission ^{µ} | | | | | | | | 1.15 |
| *Yes* | 1564 (21.3%) | 595 (44.7%) | 969 (16.1%) | 2.10 (0.07) | <0.001* | 1.78 (0.07) | <0.001* | |
| *No* | 1858 (25.3%) | 354 (26.6%) | 1504 (25.0%) | Ref. | | Ref. | | |
| *Missing* | 3923 (53.4%) | 382 (28.7%) | 3541 (58.9%) | 0.56 (0.07) | <0.001* | 1.49 (0.08) | <0.001* | |
| Biological severity of Covid-19 at admission ^{K} | | | | | | | | 1.15 |
| *Yes* | 2439 (33.2%) | 827 (62.1%) | 1612 (26.8%) | 2.28 (0.07) | <0.001* | 1.73 (0.07) | <0.001* | |
| *No* | 1861 (25.3%) | 327 (24.6%) | 1534 (25.5%) | Ref. | | Ref. | | |
| *Missing* | 3045 (41.5%) | 177 (13.3%) | 2868 (47.7%) | 0.38 (0.09) | <0.001* | 0.69 (0.10) | <0.001* | |

| | | | | | | | | |
|---|---|---|---|---|---|---|---|---|
| ^{α} Defined as having a body-mass index higher than 30 kg/m² or an International Statistical Classification of Diseases and Related Health Problems (ICD-10) diagnosis code for obesity (E66.0, E66.1, E66.2, E66.8, E66.9). ^{β} Assessed using ICD-10 codes for diabetes mellitus, diseases of the circulatory system, diseases of the respiratory system, neoplasms, and diseases of the blood and blood-forming organs and certain disorders involving the immune mechanism ((E11, J00-J99, I00-I99, C00-D49, D5-D8) based on ICD-10 classification. ^{θ} Any medication prescribed as part of clinical trials or according to compassionate use or (e.g., hydroxychloroquine, azithromycin, remdesivir, tocilizumab, or sarilumab). ^{£} Assessed using ICD-10 codes for mood and anxiety disorders (F30-F48). 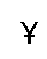 Assessed using ICD-10 codes (F00-F29 and F50-F99). ^{Ω} Included lithium or antiepileptic medications with mood stabilizing effects. ^{µ} Defined as having at least one of the following criteria: respiratory rate > 24 breaths/min or < 12 breaths/min, resting peripheral capillary oxygen saturation in ambient air < 90%, temperature > 40°C, or systolic blood pressure < 100 mm Hg. ^{K} Defined as having at least one of the following criteria: neutrophil-to-lymphocyte ratio higher than the median, *lymphocyte-to-C-reactive protein ratio* lower than the median, and plasma *lactate* levels *higher than 2 mmol*/*L.* * p-value is significant (p<0.05). | | | | | | | | |

**Table 2. Associations of any antidepressant, antidepressant classes and individual medications with the composite endpoint of death or intubation or in the full sample and in the matched analytic sample.**

| | **Full sample** | **Cox regression adjusted for age and sex** | **Multivariable (Weighted) Cox regression analysis** | **Analysis weighted by inverse-probability-weighting weights** | **Matched analytic sample** | **Univariate Cox regression in a matched analytic sample** |
|---|---|---|---|---|---|---|
| | **Number of events / Number of patients (%)** | **HR (IC95%; p-value)** | **HR (IC95%; p-value)** | **HR (IC95%; p-value)** | **Number of events / Number of patients (%)** | **HR (IC95%; p-value)** |
| *No antidepressant (n=6,885)* | 1,188 / 6,885 (17.3%) | Ref. | Ref. | Ref. | Ref. | Ref. |
| *Any antidepressant (n=460)* | 143 / 460 (31.1%) | 1.00 (0.84 - 1.20; 0.971) | 0.69 (0.57 - 0.84; <0.001*) | 0.64 (0.51 - 0.80; <0.001*) | 194/ 460 (42.2%) | 0.62 (0.50 - 0.77; <0.001*) |
| SSRIs (n=257) | 75 / 257 (29.2%) | 0.93 (0.74 - 1.17; 0.535) | 0.61 (0.47 - 0.78; <0.001*) | 0.56 (0.42 - 0.75; <0.001*) | 117 / 257 (45.5%) | 0.52 (0.39 - 0.70; <0.001*) |
| SNRIs (n=71) | 18 / 71 (25.4%) | 0.87 (0.54 - 1.40; 0.562) | 0.59 (0.68 - 0.92; 0.021*) | 0.57 (0.34 - 0.96; 0.034*) | 27 / 71 (38.0%) | 0.85 (0.32 - 1.05; 0.074) |
| Tricyclic antidepressants (n=59) | 19 / 59 (32.2%) | 1.45 (0.94 - 2.23; 0.089) | 0.96 (0.61 - 1.49; 0.850) | 0.96 (0.57 - 1.61; 0.88) | 20 / 59 (33.9%) | 0.86 (0.46 - 1.61; 0.634) |
| Tetracyclic antidepressants (n=94) | 43 / 94 (45.7%) | 1.32 (0.99 - 1.78; 0.062) | 0.86 (0.63 - 1.19; 0.368) | 0.87 (0.61 - 1.25; 0.454) | 41 / 94 (43.6%) | 0.95 (0.62 - 1.46; 0.817) |
| Presynaptic α2-antagonist antidepressant (n=44) | 12 / 44 (27.3%) | 0.73 (0.41 - 1.29; 0.280) | 0.53 (0.28 - 1.00; 0.050) | 0.29 (0.28 - 1.02; 0.058) | 16 / 44 (36.4%) | 0.64 (0.30 - 1.36; 0.249) |
| SSRIs | | | | | | |
| *Citalopram (n=27)* | 6 / 27 (22.2%) | 0.73 (0.35 - 1.54; 0.407) | 0.53 (0.27 - 1.05; 0.069) | 0.52 (0.21 - 1.29; 0.157) | 21 / 54 (38.9%) | 0.53 (0.21 - 1.33; 0.179) |
| *Escitalopram (n=84)* | 26 / 84 (31.0%) | 0.96 (0.67 - 1.40; 0.851) | 0.63 (0.43 - 0.93; 0.019*) | 0.61 (0.40 - 0.92; 0.018*) | 26 / 168 (15.5%) | 0.63 (0.40 - 0.98; 0.042*) |
| *Fluoxetine (n=35)* | 6 / 35 (17.1%) | 0.67 (0.31 - 1.47; 0.316) | 0.40 (0.19 - 0.85; 0.018*) | 0.32 (0.14 - 0.73; 0.007*) | 27 / 70 (38.6%) | 0.30 (0.12 - 0.72; 0.007*) |
| *Paroxetine (n=79)* | 27 / 79 (34.2%) | 1.08 (0.72 - 1.61; 0.703) | 0.72 (0.48 - 1.08; 0.113) | 0.77 (0.49 - 1.20; 0.251) | 76 / 158 (48.1%) | 0.62 (0.40 - 0.96; 0.034*) |
| *Sertraline (n=39)* | 13 / 39 (33.3%) | 0.94 (0.54 - 1.62; 0.817) | 0.64 (0.40 - 1.02; 0.061) | 0.59 (0.31 - 1.11; 0.103) | 32 /72 (44.4%) | 0.67 (0.35 - 1.27; 0.215) |
| *Fluvoxamine (n=1)* | 1 / 1 (100%) | NA | NA | NA | NA | NA |
| *Vortioxetine (n=3)* | 0 / 3 (0.0%) | NA | NA | NA | NA | NA |

| SNRIs | | | | | | |
|---|---|---|---|---|---|---|
| *Venlafaxine (n=49)* | 12 / 49 (24.5%) | 0.85 (0.48 - 1.50; 0.583) | 0.49 (0.29 - 0.83; 0.008*) | 0.47 (0.24 - 0.91; 0.025*) | 42 /98 (42.9%) | 0.44 (0.23 - 0.83; 0.012*) |
| *Duloxetine (n=23)* | 6 / 23 (26.1%) | 0.97 (0.42 - 2.23; 0.942) | 0.91 (0.41 - 2.02; 0.810) | 0.79 (0.34 - 1.85; 0.587) | 11 / 46 (23.9%) | 0.93 (0.34 - 2.52; 0.887) |
| *Milnacipran (n=1)* | 0 / 1 (0.0%) | NA | NA | NA | NA | NA |

| Tricyclic antidepressants | | | | | | |
|---|---|---|---|---|---|---|
| *Amitriptyline (n=47)* | 16 / 47 (34.0%) | 1.52 (0.96 - 2.40; 0.074) | 0.97 (0.59 - 1.58; 0.897) | 0.96 (0.56 - 1.65; 0.888) | 37 / 94 (39.4%) | 0.72 (0.40 - 1.30; 0.279) |
| *Clomipramine (n=12)* | 3 / 12 (25.0%) | 1.02 (0.31 - 3.33; 0.980) | 0.73 (0.26 - 2.09; 0.559) | 0.70 (0.20 - 2.38; 0.565) | 10 /24 (41.7%) | 0.63 (0.17 - 2.32 ; 0.491) |
| *Dosulepine (n=1)* | 0 / 1 (0.0%) | NA | NA | NA | NA | NA |

| Tetracyclic antidepressants | | | | | | |
|---|---|---|---|---|---|---|
| *Mianserine (n=94)* | 43 / 94 (45.7%) | 1.32 (0.99 - 1.78; 0.062) | 0.86 (0.63 - 1.19; 0.368) | 0.87 (0.61 - 1.25; 0.454) | 83 / 188 (44.1%) | 0.93 (0.65 - 1.35; 0.712) |

| α2-antagonist antidepressants | | | | | | |
|---|---|---|---|---|---|---|
| *Mirtazapine (n=44)* | 12 / 44 (27.3%) | 0.73 (0.41 - 1.29; 0.280) | 0.53 (0.28 - 1.00; 0.050) | 0.29 (0.28 - 1.02; 0.058) | 38 / 88 (43.2%) | 0.52 (0.27 - 0.99; 0.046*) |

| | | | | | | |
|---|---|---|---|---|---|---|
| * p-value is significant (p<0.05). | | | | | | |

**Table 3. Absolute dosage and fluoxetine-equivalent dosage.**

| | **Absolute dosage** | | **Fluoxetine-equivalent dosage** 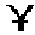 | |
|---|---|---|---|---|
| | **Mean (SE)** | **Median (SE)** | **Mean (SE)** | **Median (SE)** |
| Any antidepressant (n=460) | - | - | 16.92 (0.70) | 11.87 (0.70) |
| SSRIs (n=257) | - | - | 15.77 (0.93) | 20.00 (0.93) |
| SNRIs (n=71) | - | - | 29.77 (2.21) | 20.08 (2.21) |
| Tricyclic antidepressants (n=59) | - | - | 8.63 (1.19) | 4.91 (1.19) |
| Tetracyclic antidepressants (n=94) | - | - | 11.05 (0.67) | ¥ |
| Presynaptic α2-antagonist antidepressant (n=44) | - | - | 16.53 (1.04) | 11.79 (1.04) |

| SSRIs | | | | |
|---|---|---|---|---|
| *Citalopram (n=27)* | 15.22 (0.98) | 20.00 (0.98) | 16.91 (1.09) | 22.22 (1.09) |
| *Escitalopram (n=84)* | 11.29 (0.52) | 10.00 (0.52) | 25.08 (1.15) | 22.22 (1.15) |
| *Fluoxetine (n=35)* | 19.68 (0.81) | 20.00 (0.81) | 19.68 (0.81) | 20.00 (0.81) |
| *Paroxetine (n=79)* | 20.61 (0.76) | 20.00 (0.76) | 24.24 (0.90) | 23.53 (0.90) |
| *Sertraline (n=39)* | 67.74 (6.87) | 50.00 (6.87) | 27.51 (2.79) | 20.30 (2.79) |
| *Fluvoxamine (n=1)* | NA | NA | NA | NA |
| *Vortioxetine (n=3)* | 8.33 (3.33) | 5.00 (3.33) | 18.52 (7.41) | 11.11 (7.41) |

| SNRIs | | | | |
|---|---|---|---|---|
| *Venlafaxine (n=49)* | 98.66 (9.61) | 75.00 (9.61) | 26.41 (2.57) | 20.08 (2.57) |
| *Duloxetine (n=23)* | 55.00 (5.78) | 60.00 (5.78) | 36.67 (3.85) | 40.00 (3.85) |
| *Milnacipran (n=1)* | 50 (NA) | 50 (NA) | 20 (NA) | 20 (NA) |

| Tricyclic antidepressants | | | | |
|---|---|---|---|---|
| *Amitriptyline (n=47)* | 26.35 (4.37) | 15.00 (4.37) | 8.62 (1.43) | 4.91 (1.73) |
| *Clomipramine (n=12)* | 35.00 (12.56) | 18.75 (12.56) | 12.01 (4.33) | 6.46 (4.33) |
| *Dosulepine (n=1)* | 50.00 (NA) | 50.00 (NA) | 17.78 (NA) | 17.78 (NA) |

| Tetracyclic antidepressant | | | | |
|---|---|---|---|---|
| *Mianserine (n=94)* | 27.93 (1.69) | 30.00 (1.69) | 11.05 (0.67) | 11.87 (0.67) |
| Presynaptic α2-antagonist antidepressant | | | | |
| *Mirtazapine (n=44)* | 21.04 (1.32) | 15.00 (1.32) | 16.53 (1.04) | 11.79 (1.04) |

| | | | | |
|---|---|---|---|---|
| 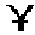 Individual antidepressant medication dosage were converted into fluoxetine-equivalent dosage based on prior work.²⁷ | | | | |

**Table 4. Time to follow-up.**

| | **Range** | **Mean (SE)** | **Median (SE)** |
|---|---|---|---|
| No antidepressant (n=6,885) | 1 - 117 | 18.40 (0.33) | 4 (0.33) |
| Any antidepressant (n=460) | 1 - 112 | 20.11 (1.08) | 11 (1.08) |
| SSRIs (n=257) | 1 - 111 | 21.99 (1.56) | 11 (1.56) |
| SNRIs (n=71) | 1 - 112 | 18.82 (2.82) | 10 (2.82) |
| Tricyclic antidepressants (n=59) | 1 - 112 | 17.51 (2.95) | 8 (2.95) |
| Tetracyclic antidepressants (n=94) | 1 - 76 | 16.94 (1.88) | 11 (1.88) |
| Presynaptic α2-antagonist antidepressant (n=44) | 1 - 83 | 25.73 (3.80) | 15 (3.80) |

| SSRIs | | | |
|---|---|---|---|
| *Citalopram (n=27)* | 1 - 71 | 15.15 (3.50) | 9 (3.50) |
| *Escitalopram (n=84)* | 1 - 103 | 22.50 (2.60) | 13 (2.60) |
| *Fluoxetine (n=35)* | 1 - 105 | 26.77 (4.56) | 15 (4.56) |
| *Paroxetine (n=79)* | 1 - 105 | 20.92 (2.78) | 8 (2.78) |
| *Sertraline (n=39)* | 1 - 111 | 24.26 (4.73) | 12 (4.73) |
| *Fluvoxamine (n=1)* | | 18.00 (NA) | 18 (NA) |
| *Vortioxetine (n=3)* | 7 - 49 | 21.00 (14.00) | 7 (14.00) |

| SNRIs | | | |
|---|---|---|---|
| *Venlafaxine (n=49)* | 1 - 105 | 17.88 (3.14) | 11 (3.14) |
| *Duloxetine (n=23)* | 1 - 112 | 22.00 (5.89) | 8 (5.89) |
| *Milnacipran (n=1)* | | 54.00 (NA) | 54 (NA) |
| Tricyclic antidepressants | | | |
| *Amitriptyline (n=47)* | 1 - 112 | 18.47 (3.45) | 9 (3.45) |
| *Clomipramine (n=12)* | 1 - 70 | 14.58 (5.31) | 9 (5.31) |
| *Dosulepine (n=1)* | | 1.00 (NA) | 1 (NA) |
| Tetracyclic antidepressant | | | |
| *Mianserine (n=94)* | 1 - 76 | 16.94 (1.88) | 11 (1.88) |
| Presynaptic α2-antagonist antidepressant | | | |
| *Mirtazapine (n=44)* | 1 - 83 | 22.73 (3.80) | 15.5 (3.80) |

**Table 5. Characteristics of patients receiving or not receiving antidepressants in the matched and unmatched analytic samples.**

| | Exposed to any antidepressant N = 460 | Not exposed to any antidepressant N = 6,885 | Non-exposed matched group N = 460 | Exposed to any antidepressant vs. Not exposed to any antidepressant | Exposed to any antidepressant vs. Not exposed to antidepressants | Exposed to any antidepressant vs. Non-exposed matched group |
|---|---|---|---|---|---|---|
| | | | | Crude analysis | Analysis weighted by inverse-probability-weighting weights | Matched analytic sample analysis |
| | N (%) | N (%) | N (%) | Chi-square test (p-value) | Weighted Chi-square test (p-value) | Chi-square test (p-value) |
| *Characteristics* Age (years) | | | | 418.55 (<0.001*) | 265.26 (<0.001*) | 3.21 (0.361) |
| *18-50* | 33 (7.2%) | 2,676 (38.9%) | 39 (8.5%) | | | |
| *51-70* | 120 (26.1%) | 2,410 (35.0%) | 140 (30.4%) | | | |
| *71-80* | 101 (22.0%) | 841 (12.2%) | 94 (20.4%) | | | |
| *81+* | 206 (44.8%) | 958 (13.9%) | 187 (40.7%) | | | |
| Sex | | | | 16.25 (<0.001*) | 19.01 (<0.001*) | 4.78 (0.029*) |
| *Women* | 269 (58.5%) | 3,350 (48.7%) | 235 (51.1%) | | | |
| *Men* | 191 (41.5%) | 3,535 (51.3%) | 225 (48.9%) | | | |
| Obesity ^{α} | | | | 41.59 (<0.001*) | 26.78 (<0.01*) | 3.20 (0.074) |
| *Yes* | 107 (23.3%) | 868 (12.6%) | 84 (18.3%) | | | |
| *No* | 353 (76.7%) | 6,017 (87.4%) | 376 (81.7%) | | | |
| Smoking | | | | 16.48 (<0.001*) | 5.82 (0.016)* | 0.24 (0.625) |
| *Yes* | 63 (13.7%) | 560 (8.1%) | 57 (12.4%) | | | |
| *No* | 397 (86.3%) | 6,325 (91.9%) | 403 (87.6%) | | | |
| Any medical condition ^{β} | | | | 92.52 (<0.001*) | 33.24 (<0.001*) | 2.80 (0.094) |
| *Yes* | 257 (55.9%) | 2,317 (33.7%) | 283 (61.5%) | | | |
| *No* | 203 (44.1%) | 4,568 (66.3%) | 177 (38.5%) | | | |
| Medication according to compassionate use or as part of a clinical trial ^{θ} | | | | 8.62 (<0.001*) | 2.32 (0.128) | 0.01 (0.936) |
| *Yes* | 101 (22.0%) | 1,139 (16.5%) | 99 (21.5%) | | | |
| *No* | 359 (78.0%) | 5,746 (83.5%) | 361 (78.5%) | | | |
| Any current mood or anxiety disorder ^{£} | | | | 229.32 (<0.001*) | 444.46 (<0.001*) | <0.01 (>0.99) |
| *Yes* | 49 (10.7%) | 76 (1.1%) | 49 (10.7%) | | | |
| *No* | 411 (89.3%) | 6,809 (98.9%) | 411 (89.3%) | | | |
| Any other current psychiatric disorder 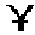 | | | | 143.00 (<0.001*) | 124.72 (<0.001*) | 0.01 (0.926) |
| *Yes* | 69 (15.0%) | 235 (3.4%) | 67 (14.6%) | | | |
| *No* | 391 (85.0%) | 6,650 (96.6%) | 393 (85.4%) | | | |
| Any benzodiazepine or Z-drug | | | | 924.52 (<0.001*) | 1094.87 (<0.001*) | 0.43 (0.51) |
| *Yes* | 239 (52.0%) | 513 (7.5%) | 228 (49.6%) | | | |
| *No* | 221 (48.0%) | 6,372 (92.5%) | 232 (50.4%) | | | |
| Any mood stabilizer medication ^{Ω} | | | | 400.52 (<0.001*) | 560.21 (<0.001*) | 0.10 (0.746) |
| *Yes* | 99 (21.5%) | 188 (2.7%) | 94 (20.4%) | | | |
| *No* | 361 (78.5%) | 6,697 (97.3%) | 366 (79.6%) | | | |
| Any antipsychotic medication | | | | 366.17 (<0.001*) | 566.00 (<0.001*) | <0.01 (>0.99) |
| *Yes* | 91 (19.8%) | 173 (2.51%) | 91 (19.8%) | | | |
| *No* | 369 (80.2%) | 6,712 (97.5%) | 369 (80.2%) | | | |
| Clinical severity of Covid-19 at admission ^{µ} | | | | 133.78 (<0.001*) | 59.49 (<0.001*) | 0.15 (0.927) |
| *Yes* | 149 (32.4%) | 1,415 (20.6%) | 154 (33.5%) | | | |
| *No* | 185 (40.2%) | 1,673 (24.3%) | 180 (39.1%) | | | |
| *Missing* | 126 (27.4%) | 3,797 (55.1%) | 126 (27.4%) | | | |
| Biological severity of Covid-19 at admission ^{K} | | | | 148.70 (<0.001*) | 61.11 (<0.001*) | 0.38 (0.829) |
| *Yes* | 226 (49.1%) | 2,213 (32.1%) | 235 (51.1%) | | | |
| *No* | 168 (36.5%) | 1,693 (24.6%) | 163 (35.4%) | | | |
| *Missing* | 66 (14.3%) | 2,979 (43.3%) | 62 (13.5%) | | | |

| | | | | | | |
|---|---|---|---|---|---|---|
| ^{α} Defined as having a body-mass index higher than 30 kg/m² or an International Statistical Classification of Diseases and Related Health Problems (ICD-10) diagnosis code for obesity (E66.0, E66.1, E66.2, E66.8, E66.9). ^{β} Assessed using ICD-10 codes for diabetes mellitus, diseases of the circulatory system, diseases of the respiratory system, neoplasms, and diseases of the blood and blood-forming organs and certain disorders involving the immune mechanism ((E11, J00-J99, I00-I99, C00-D49, D5-D8) based on ICD-10 classification. ^{θ} Any medication prescribed as part of clinical trials or according to compassionate use or (e.g., hydroxychloroquine, azithromycin, remdesivir, tocilizumab, or sarilumab). ^{£} Assessed using ICD-10 codes for mood and anxiety disorders (F30-F48). ^{¥} Assessed using ICD-10 codes (F00-F29 and F50-F99). ^{Ω} Included lithium or antiepileptic medications with mood stabilizing effects. ^{µ} Defined as having at least one of the following criteria: respiratory rate > 24 breaths/min or < 12 breaths/min, resting peripheral capillary oxygen saturation in ambient air < 90%, temperature > 40°C, or systolic blood pressure < 100 mm Hg. ^{K} Defined as having at least one of the following criteria: neutrophil-to-lymphocyte ratio higher than the median, *lymphocyte-to-C-reactive protein ratio* lower than the median, and plasma *lactate* levels *higher than 2 mmol*/*L.* * p-value is significant (p<0.05). | | | | | | |

**Table 6. Characteristics of patients receiving SSRIs or not receiving any antidepressant in the matched and unmatched analytic samples.**

| | Exposed to any SSRI N=275 | Not exposed to any antidepressan t N = 6,885 | Non-exposed matched group N = 275 | Exposed to any SSRI vs. Not exposed to any antidepressant | Exposed to any SSRI vs. Not exposed to any antidepressant | Exposed to any SSRI vs. Non-exposed matched group |
|---|---|---|---|---|---|---|
| | | | | Crude analysis | Analysis weighted by inverse-probability-weighting weights | Matched analytic sample analysis |
| | N (%) | N (%) | N (%) | Chi-square test (p-value) | Weighted Chi-square test (p-value) | Chi-square test (p-value) |
| *Characteristics* Age (years) | | | | 231.83 (<0.001*) | 99.08 (<0.001*) | 0.55 (0.908) |
| *18-50* | 22 (8.6%) | 2,676 (38.9%) | 24 (9.34%) | | | |
| *51-70* | 64 (24.9%) | 2,410 (35.0%) | 70 (27.2%) | | | |
| *71-80* | 61 (23.7%) | 841 (12.2%) | 58 (22.6%) | | | |
| *81+* | 110 (42.8%) | 958 (13.9%) | 105 (40.9%) | | | |
| Sex | | | | 13.90 (<0.001*) | 8.53 (0.003*) | 2.57 (0.109) |
| *Women* | 156 (60.7%) | 3,350 (48.7%) | 137 (53.3%) | | | |
| *Men* | 101 (39.3%) | 3,535 (51.3%) | 120 (46.7%) | | | |
| Obesity ^{α} | | | | 29.94 (<0.001*) | 15.62 (<0.001*) | 0.63 (0.201) |
| *Yes* | 63 (24.5%) | 868 (12.6%) | 50 (19.5%) | | | |
| *No* | 194 (75.5%) | 6,017 (87.4%) | 207 (80.5%) | | | |
| Smoking | | | | 9.06 (0.003*) | 2.99 (0.084) | 0.9 (0.343) |
| *Yes* | 35 (13.6%) | 560 (8.1%) | 27 (10.5%) | | | |
| *No* | 222 (86.4%) | 6,325 (91.9%) | 230 (89.5%) | | | |
| Any medical condition ^{β} | | | | 44.94 (<0.001*) | 10.64 (0.001*) | 2.3 (0.129) |
| *Yes* | 139 (54.1%) | 2,317 (33.7%) | 157 (61.1%) | | | |
| *No* | 118 (45.9%) | 4,568 (66.3%) | 100 (38.9%) | | | |
| Medication according | | | | | | |
| to compassionate use or as part of a clinical trial ^{θ} | | | | 1.71 (0.190) | 0.03 (0.867) | <0.01 (>.99) |
| *Yes* | 51 (19.8%) | 1,139 (16.5%) | 51 (19.8%) | | | |
| *No* | 206 (80.2%) | 5,746 (83.5%) | 206 (80.2%) | | | |
| Any current mood or anxiety disorder ^{£} | | | | 147.54 (<0.001*) | 195.76 (<0.001*) | <0.01 (>.99) |
| *Yes* | 27 (10.5%) | 76 (1.1%) | 27 (10.5%) | | | |
| *No* | 230 (89.5%) | 6,809 (98.9%) | 230 (89.5%) | | | |
| Any other current psychiatric disorder 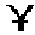 | | | | 107.65 (<0.001*) | 70.38 (<0.001*) | <0.01 (>.99) |
| *Yes* | 42 (16.3%) | 235 (3.4%) | 42 (16.3%) | | | |
| *No* | 215 (83.7%) | 6,650 (96.6%) | 215 (83.7%) | | | |
| Any benzodiazepine or Z-drug | | | | 585.63 (<0.001*) | 477.45 (<0.001*) | 0.19 (0.659) |
| *Yes* | 133 (51.8%) | 513 (7.5%) | 127 (49.4%) | | | |
| *No* | 124 (48.2%) | 6,372 (92.5%) | 130 (50.6%) | | | |
| Any mood stabilizer medication ^{Ω} | | | | 201.01 (<0.001*) | 195.76 (<0.001*) | 0.01 (0.91) |
| *Yes* | 49 (19.1%) | 188 (2.7%) | 47 (18.3%) | | | |
| *No* | 208 (80.9%) | 6,697 (97.3%) | 210 (81.7%) | | | |
| Any antipsychotic medication | | | | 249.21 (<0.001*) | 277.47 (<0.001*) | <0.01 (>0.99) |
| *Yes* | 52 (20.2%) | 173 (2.51%) | 52 (20.2%) | | | |
| *No* | 205 (79.8%) | 6,712 (97.5%) | 205 (79.8%) | | | |
| Clinical severity of Covid-19 at admission ^{µ} | | | | 65.79 (<0.001*) | 21.13 (<0.001*) | 0.23 (0.890) |
| *Yes* | 79 (30.7%) | 1,415 (20.6%) | 84 (32.7%) | | | |
| *No* | 102 (39.7%) | 1,673 (24.3%) | 100 (38.9%) | | | |
| *Missing* | 76 (29.6%) | 3,797 (55.1%) | 73 (28.4%) | | | |
| Biological severity of Covid-19 at admission ^{K} | | | | 89.11 (<0.001*) | 26.15 (<0.001*) | 0.4 (0.821) |
| *Yes* | 116 (45.1%) | 2,213 (32.1%) | 123 (47.9%) | | | |
| *No* | 105 (40.9%) | 1,693 (24.6%) | 99 (38.5%) | | | |
| *Missing* | 36 (14.0%) | 2,979 (43.3%) | 35 (13.6%) | | | |

| | | | | | | |
|---|---|---|---|---|---|---|
| ^{α} Defined as having a body-mass index higher than 30 kg/m² or an International Statistical Classification of Diseases and Related Health Problems (ICD-10) diagnosis code for obesity (E66.0, E66.1, E66.2, E66.8, E66.9). ^{β} Assessed using ICD-10 codes for diabetes mellitus, diseases of the circulatory system, diseases of the respiratory system, neoplasms, and diseases of the blood and blood-forming organs and certain disorders involving the immune mechanism ((E11, J00-J99, I00-I99, C00-D49, D5-D8) based on ICD-10 classification. ^{θ} Any medication prescribed as part of clinical trials or according to compassionate use or (e.g., hydroxychloroquine, azithromycin, remdesivir, tocilizumab, or sarilumab). ^{£} Assessed using ICD-10 codes for mood and anxiety disorders (F30-F48). 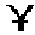 Assessed using ICD-10 codes (F00-F29 and F50-F99). ^{Ω} Included lithium or antiepileptic medications with mood stabilizing effects. ^{µ} Defined as having at least one of the following criteria: respiratory rate > 24 breaths/min or < 12 breaths/min, resting peripheral capillary oxygen saturation in ambient air < 90%, temperature > 40°C, or systolic blood pressure < 100 mm Hg. ^{K} Defined as having at least one of the following criteria: neutrophil-to-lymphocyte ratio higher than the median, *lymphocyte-to-C-reactive protein ratio* lower than the median, and plasma *lactate* levels *higher than 2 mmol*/*L.* * p-value is significant (p<0.05). | | | | | | |

**Table 7. Characteristics of patients receiving SNRIs or not receiving any antidepressant in the matched and unmatched analytic samples.**

| | Exposed to any SNRI N=71 | Not exposed to any antidepressa nt N = 6,885 | Non-exposed matched group N = 71 | Exposed to any SNRI vs. Not exposed to any antidepressant | Exposed to any SNRI vs. Not exposed to any antidepressant | Exposed to any SNRI vs. Non-exposed matched group |
|---|---|---|---|---|---|---|
| | | | | Crude analysis | Analysis weighted by inverse-probability-weighting weights | Matched analytic sample analysis |
| | N (%) | N (%) | N (%) | Chi-square test (p-value) | Weighted Chi-square test (p-value) | Chi-square test (p-value) |
| *Characteristics* Age (years) | | | | 20.71 (<0.001*) | 8.79 (0.032*) | 0.78 (0.855) |
| *18-50* | 3 (4.23%) | 2,676 | 5 (7.04%) | | | |
| *51-70* | 27 (38.0%) | 2,410 | 25 (35.2%) | | | |
| *71-80* | 16 (22.5%) | 841 (12.2%) | 18 (25.4%) | | | |
| 81+ | 25 (35.2%) | 958 (13.9%) | 23 (32.4%) | | | |
| Sex | | | | 3.53 (0.060) | 3.42 (0.064) | 0.46 (0.498) |
| *Women* | 43 (60.6%) | 3,350 | 38 (53.5%) | | | |
| *Men* | 28 (39.4%) | 3,535 | 33 (46.5%) | | | |
| Obesity ^{α} | | | | 11.41 (<0.001*) | 1.93 (0.165) | 0.63 (0.427) |
| *Yes* | 19 (26.8%) | 868 (12.6%) | 14 (19.7%) | | | |
| *No* | 52 (73.2%) | 6,017 | 57 (80.3%) | | | |
| Smoking | | | | 13.98 (<0.001*) | 2.71 (0.100) | 0.04 (0.833) |
| *Yes* | 15 (21.1%) | 560 (8.1%) | 13 (18.3%) | | | |
| *No* | 56 (78.9%) | 6,325 | 58 (81.7%) | | | |
| Any medical condition ^{β} | | | | 19.27 (<0.001*) | 1.71 (0.190) | 0.48 (0.488) |
| *Yes* | 42 (59.2%) | 2,317 | 47 (66.2%) | | | |
| *No* | 29 (40.8%) | 4,568 | 24 (33.8%) | | | |
| Medication according to compassionate use or as part of a clinical trial ^{θ} | | | | 0.76 (0.383) | 0.05 (0.829) | <0.01 (>0.99) |
| *Yes* | 15 (21.1%) | 1,139 | 14 (19.7%) | | | |
| *No* | 56 (78.9%) | 5,746 | 57 (80.3%) | | | |
| Any current mood or anxiety disorder ^{£} | | | | 128.04 (<0.001*) | 102.27 (<0.001*) | <0.01 (>0.99) |
| *Yes* | 12 (16.9%) | 76 (1.1%) | 12 (16.9%) | | | |
| *No* | 59 (83.1%) | 6,809 | 59 (83.1%) | | | |
| Any other current psychiatric disorder 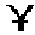 | | | | 20.51 (<0.001*) | 0.98 (0.322) | <0.01 (>0.99) |
| *Yes* | 10 (14.1%) | 235 (3.4%) | 10 (14.1%) | | | |
| *No* | 61 (85.9%) | 6,650 | 61 (85.9%) | | | |
| Any benzodiazepine or Z-drug | | | | 198.23 (<0.001*) | 52.64 (<0.001*) | 0.03 (0.867) |
| *Yes* | 38 (53.5%) | 513 (7.5%) | 36 (50.7%) | | | |
| *No* | 33 (46.5%) | 6,372 (92.5%) | 35 (49.3%) | | | |
| Any mood stabilizer medication ^{Ω} | | | | 164.71 (<0.001*) | 91.98 (<0.001*) | <0.01 (>0.99) |
| *Yes* | 21 (29.6%) | 188 (2.7%) | 21 (29.6%) | | | |
| *No* | 50 (70.4%) | 6,697 (97.3%) | 50 (70.4%) | | | |
| Any antipsychotic medication | | | | 73.08 (<0.001*) | 47.44 (<0.001*) | <0.01 (>0.99) |
| *Yes* | 14 (19.7%) | 173 (2.5%) | 14 (19.7%) | | | |
| *No* | 57 (80.3%) | 6,712 (97.5%) | 57 (80.3%) | | | |
| Clinical severity of Covid-19 at admission ^{µ} | | | | 24.20 (<0.001*) | 2.95 (0.229) | 0.32 (0.853) |
| *Yes* | 18 (25.4%) | 1,415 (20.6%) | 21 (29.6%) | | | |
| *No* | 33 (46.5%) | 1,673 (24.3%) | 31 (43.7%) | | | |
| *Missing* | 20 (28.2%) | 3,797 (55.1%) | 19 (26.8%) | | | |
| Biological severity of Covid-19 at admission ^{K} | | | | 30.45 (<0.001*) | 4.70 (0.095) | 0.07 (0.965) |
| *Yes* | 39 (54.9%) | 2,213 (32.1%) | 38 (53.5%) | | | |
| *No* | 24 (33.8%) | 1,693 (24.6%) | 24 (33.8%) | | | |
| *Missing* | 8 (11.3%) | 2,979 (43.3%) | 9 (12.7%) | | | |

| | | | | | | |
|---|---|---|---|---|---|---|
| ^{α} Defined as having a body-mass index higher than 30 kg/m² or an International Statistical Classification of Diseases and Related Health Problems (ICD-10) diagnosis code for obesity (E66.0, E66.1, E66.2, E66.8, E66.9). ^{β} Assessed using ICD-10 codes for diabetes mellitus, diseases of the circulatory system, diseases of the respiratory system, neoplasms, and diseases of the blood and blood-forming organs and certain disorders involving the immune mechanism ((E11, J00-J99, I00-I99, C00-D49, D5-D8) based on ICD-10 classification. ^{θ} Any medication prescribed as part of clinical trials or according to compassionate use or (e.g., hydroxychloroquine, azithromycin, remdesivir, tocilizumab, or sarilumab). ^{£} Assessed using ICD-10 codes for mood and anxiety disorders (F30-F48). 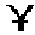 Assessed using ICD-10 codes (F00-F29 and F50-F99). ^{Ω} Included lithium or antiepileptic medications with mood stabilizing effects. ^{µ} Defined as having at least one of the following criteria: respiratory rate > 24 breaths/min or < 12 breaths/min, resting peripheral capillary oxygen saturation in ambient air < 90%, temperature > 40°C, or systolic blood pressure < 100 mm Hg. ^{K} Defined as having at least one of the following criteria: neutrophil-to-lymphocyte ratio higher than the median, *lymphocyte-to-C-reactive protein ratio* lower than the median, and plasma *lactate* levels *higher than 2 mmol*/*L.* * p-value is significant (p<0.05). | | | | | | |

**Table 8. Characteristics of patients receiving tricyclic antidepressants or not receiving any antidepressant in the matched and unmatched analytic samples.**

| | Exposed to any tricyclic antidepressant N=59 | Not exposed to any antidepressant N = 6,885 | Non-exposed matched group N = 59 | Exposed to any tricyclic antidepressant vs. Not exposed to any antidepressant | Exposed to any tricyclic antidepressant vs. Not exposed to any antidepressant | Exposed to any tricyclic antidepressant vs. Non-exposed matched group |
|---|---|---|---|---|---|---|
| | | | | Crude analysis | Analysis weighted by inverse-probability-weighting weights | Matched analytic sample analysis |
| | N (%) | N (%) | N (%) | Chi-square test (p-value) | Weighted Chi-square test (p-value) | Chi-square test (p-value) |
| *Characteristics* Age (years) | | | | 23.51 (<0.001*) | 2.63 (0.453) | 0.96 (0.810) |
| *18-50* | 8 (13.6%) | 2,676 (38.9%) | 8 (13.6%) | | | |
| *51-70* | 21 (35.6%) | 2,410 (35.0%) | 25 (42.4%) | | | |
| *71-80* | 15 (25.4%) | 841 (12.2%) | 11 (18.6%) | | | |
| 81+ | 15 (25.4%) | 958 (13.9%) | 15 (25.4%) | | | |
| Sex | | | | 9.35 (0.002*) | 3.01 (0.083*) | 2.89 (0.089) |
| *Women* | 41 (69.5%) | 3,350 (48.7%) | 31 (52.5%) | | | |
| *Men* | 18 (30.5%) | 3,535 (51.3%) | 28 (47.5%) | | | |
| Obesity ^{α} | | | | 29.72 (<0.001*) | 9.34 (0.002*) | 1.42 (0.234) |
| *Yes* | 22 (37.3%) | 868 (12.6%) | 15 (25.4%) | | | |
| *No* | 37 (62.7%) | 6,017 (87.4%) | 44 (74.6%) | | | |
| Smoking | | | | 0.65 (0.422) | 0.02 (0.888) | <0.01 (>0.99) |
| *Yes* | 7 (11.9%) | 560 (8.1%) | 6 (10.2%) | | | |
| *No* | 52 (88.1%) | 6,325 (91.9%) | 53 (89.8%) | | | |
| Any medical condition ^{β} | | | | 16.18 (<0.001*) | 2.50 (0.114) | 0.04 (0.85) |
| *Yes* | 35 (59.3%) | 2,317 (33.7%) | 37 (62.7%) | | | |
| *No* | 24 (40.7%) | 4,568 (66.3%) | 22 (37.3%) | | | |
| Medication according to compassionate use or as part of a clinical trial ^{θ} | | | | 7.24 (0.007*) | 0.84 (0.360) | <0.01 (>0.99) |
| *Yes* | 18 (30.5%) | 1,139 (16.5%) | 18 (30.5%) | | | |
| *No* | 41 (69.5%) | 5,746 (83.5%) | 41 (69.5%) | | | |
| Any current mood or anxiety disorder ^{£} | | | | 85.53 (<0.001*) | 51.14 (<0.001*) | <0.01 (>0.99) |
| *Yes* | 9 (15.3%) | 76 (1.1%) | 9 (15.3%) | | | |
| *No* | 50 (84.7%) | 6,809 (98.9%) | 50 (84.7%) | | | |
| Any other current psychiatric disorder 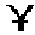 | | | | 3.10 (0.078) | 1.16 (0.281) | <0.01 (>0.99) |
| *Yes* | 5 (8.47%) | 235 (3.4%) | 5 (8.47%) | | | |
| *No* | 54 (91.5%) | 6,650 (96.6%) | 54 (91.5%) | | | |
| Any benzodiazepine or Z-drug | | | | 85.92 (<0.001*) | 21.78 (<0.001*) | <0.01 (>0.99) |
| *Yes* | 24 (40.7%) | 513 (7.5%) | 23 (39.0%) | | | |
| *No* | 35 (59.3%) | 6,372 (92.5%) | 36 (61.0%) | | | |
| Any mood stabilizer medication ^{Ω} | | | | 205.30 (<0.001*) | 68.36 (<0.001*) | <0.01 (>0.99) |
| *Yes* | 21 (35.6%) | 188 (2.7%) | 21 (35.6%) | | | |
| *No* | 38 (64.4%) | 6,697 (97.3%) | 38 (64.4%) | | | |
| Any antipsychotic medication | | | | 92.55 (<0.001*) | 36.68 (<0.001*) | <0.01 (>0.99) |
| *Yes* | 14 (23.7%) | 173 (2.5%) | 14 (23.7%) | | | |
| *No* | 45 (76.3%) | 6,712 (97.5%) | 45 (76.3%) | | | |
| Clinical severity of Covid-19 at admission ^{µ} | | | | 26.03 (<0.001*) | 3.95 (0.138) | 0.7 (0.706) |
| *Yes* | 22 (37.3%) | 1,415 (20.6%) | 23 (39.0%) | | | |
| *No* | 24 (40.7%) | 1,673 (24.3%) | 20 (33.9%) | | | |
| *Missing* | 13 (22.0%) | 3,797 (55.1%) | 16 (27.1%) | | | |
| Biological severity of Covid-19 at admission ^{K} | | | | 15.41 (<0.001*) | 1.12 (0.572) | 1.17 (0.566) |
| *Yes* | 30 (50.8%) | 2,213 (32.1%) | 34 (57.6%) | | | |
| *No* | 18 (30.5%) | 1,693 (24.6%) | 18 (30.5%) | | | |
| *Missing* | 11 (18.6%) | 2,979 (43.3%) | 7 (11.9%) | | | |

| | | | | | | |
|---|---|---|---|---|---|---|
| ^{α} Defined as having a body-mass index higher than 30 kg/m² or an International Statistical Classification of Diseases and Related Health Problems (ICD-10) diagnosis code for obesity (E66.0, E66.1, E66.2, E66.8, E66.9). ^{β} Assessed using ICD-10 codes for diabetes mellitus, diseases of the circulatory system, diseases of the respiratory system, neoplasms, and diseases of the blood and blood-forming organs and certain disorders involving the immune mechanism ((E11, J00-J99, I00-I99, C00-D49, D5-D8) based on ICD-10 classification. ^{θ} Any medication prescribed as part of clinical trials or according to compassionate use or (e.g., hydroxychloroquine, azithromycin, remdesivir, tocilizumab, or sarilumab). ^{£} Assessed using ICD-10 codes for mood and anxiety disorders (F30-F48). 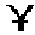 Assessed using ICD-10 codes (F00-F29 and F50-F99). ^{Ω} Included lithium or antiepileptic medications with mood stabilizing effects. ^{µ} Defined as having at least one of the following criteria: respiratory rate > 24 breaths/min or < 12 breaths/min, resting peripheral capillary oxygen saturation in ambient air < 90%, temperature > 40°C, or systolic blood pressure < 100 mm Hg. ^{K} Defined as having at least one of the following criteria: neutrophil-to-lymphocyte ratio higher than the median, *lymphocyte-to-C-reactive protein ratio* lower than the median, and plasma *lactate* levels *higher than 2 mmol*/*L.* * p-value is significant (p<0.05). | | | | | | |

**Table 9. Characteristics of patients receiving tetracyclic antidepressants or not receiving any antidepressant in the matched and unmatched analytic samples.**

| | Exposed to any tetracyclic antidepressant N=94 | Not exposed to any antidepressant N = 6,885 | Non-exposed matched group N = 94 | Exposed to any tetracyclic antidepressant vs. Not exposed to any antidepressant | Exposed to any tetracyclic antidepressant vs. Not exposed to any antidepressant | Exposed to any tetracyclic antidepressant vs. Non-exposed matched group |
|---|---|---|---|---|---|---|
| | | | | Crude analysis | Analysis weighted by inverse-probability-weighting weights | Matched analytic sample analysis |
| | N (%) | N (%) | N (%) | Chi-square test (p-value) | Weighted Chi-square test (p-value) | Chi-square test (p-value) |
| *Characteristics* Age (years) | | | | 236.19 (<0.001) | 49.74 (<0.001*) | 1.1 (0.776) |
| *18-50* | 1 (1.1%) | 2,676 (38.9%) | 1 (1.1%) | | | |
| *51-70* | 12 (12.8%) | 2,410 (35.0%) | 16 (17.0%) | | | |
| *71-80* | 17 (18.1%) | 841 (12.2%) | 13 (13.8%) | | | |
| 81+ | 64 (68.1%) | 958 (13.9%) | 64 (68.1%) | | | |
| Sex | | | | 0.32 (0.572) | 0.25 (0.617) | 1.06 (0.303) |
| *Women* | 49 (52.1%) | 3,350 (48.7%) | 57 (60.6%) | | | |
| *Men* | 45 (47.9%) | 3,535 (51.3%) | 37 (39.4%) | | | |
| Obesity ^{α} | | | | 0.04 (0.843) | <0.01 (0.989) | 0.05 (0.827) |
| *Yes* | 13 (13.8%) | 868 (12.6%) | 11 (11.7%) | | | |
| *No* | 81 (86.2%) | 6,017 (87.4%) | 83 (88.3%) | | | |
| Smoking | | | | 0.48 (0.849) | 0.06 (0.805) | 0.26 (0.611) |
| *Yes* | 10 (10.6%) | 560 (8.1%) | 7 (7.45%) | | | |
| *No* | 84 (89.4%) | 6,325 (91.9%) | 87 (92.6%) | | | |
| Any medical condition ^{β} | | | | 31.26 (<0.001) | 4.86 (0.028*) | 0.37 (0.542) |
| *Yes* | 58 (61.7%) | 2,317 (33.7%) | 63 (67.0%) | | | |
| *No* | 36 (38.3%) | 4,568 (66.3%) | 31 (33.0%) | | | |
| Medication according to compassionate use or as part of a clinical trial ^{θ} | | | | 1.18 (0.278) | 0.28 (0.599) | <0.01 (>0.99) |
| *Yes* | 20 (21.3%) | 1,139 (16.5%) | 20 (21.3%) | | | |
| *No* | 74 (78.7%) | 5,746 (83.5%) | 74 (78.7%) | | | |
| Any current mood or anxiety disorder ^{£} | | | | 48.49 (<0.001) | 23.83 (<0.001*) | <0.01 (>0.99) |
| *Yes* | 9 (9.57%) | 76 (1.1%) | 9 (9.57%) | | | |
| *No* | 85 (90.4%) | 6,809 (98.9%) | 85 (90.4%) | | | |
| Any other current psychiatric disorder 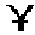 | | | | 53.22 (<0.001) | 16.73 (<0.001*) | <0.01 (>0.99) |
| *Yes* | 17 (18.1%) | 235 (3.4%) | 17 (18.1%) | | | |
| *No* | 77 (81.9%) | 6,650 (96.6%) | 77 (81.9%) | | | |
| Any benzodiazepine or Z-drug | | | | 356.13 (<0.001) | 133.93 (<0.001*) | 0.02 (0.881) |
| *Yes* | 58 (61.7%) | 513 (7.5%) | 56 (59.6%) | | | |
| *No* | 36 (38.3%) | 6,372 (92.5%) | 38 (40.4%) | | | |
| Any mood stabilizer medication ^{Ω} | | | | 36.97 (<0.001) | 3.33 (0.068) | <0.01 (>0.99) |
| *Yes* | 13 (13.8%) | 188 (2.7%) | 13 (13.8%) | | | |
| *No* | 81 (86.2%) | 6,697 (97.3%) | 81 (86.2%) | | | |
| Any antipsychotic medication | | | | 127.67 (<0.001) | 76.68 (<0.001*) | <0.01 (>0.99) |
| *Yes* | 21 (22.3%) | 173 (2.5%) | 21 (22.3%) | | | |
| *No* | 73 (77.7%) | 6,712 (97.5%) | 73 (77.7%) | | | |
| Clinical severity of Covid-19 at admission ^{µ} | | | | 46.31 (<0.001) | 8.25 (0.016) | 0.20 (0.906) |
| *Yes* | 37 (39.4%) | 1,415 (20.6%) | 35 (37.2%) | | | |
| *No* | 38 (40.4%) | 1,673 (24.3%) | 41 (43.6%) | | | |
| *Missing* | 19 (20.2%) | 3,797 (55.1%) | 18 (19.1%) | | | |
| Biological severity of Covid-19 at admission ^{K} | | | | 30.69 (<0.001) | 4.71 (0.095) | 0.41 (0.816) |
| *Yes* | 47 (50.0%) | 2,213 (32.1%) | 43 (45.7%) | | | |
| *No* | 33 (35.1%) | 1,693 (24.6%) | 37 (39.4%) | | | |
| *Missing* | 14 (14.9%) | 2,979 (43.3%) | 14 (14.9%) | | | |

| | | | | | | |
|---|---|---|---|---|---|---|
| ^{α} Defined as having a body-mass index higher than 30 kg/m² or an International Statistical Classification of Diseases and Related Health Problems (ICD-10) diagnosis code for obesity (E66.0, E66.1, E66.2, E66.8, E66.9). ^{β} Assessed using ICD-10 codes for diabetes mellitus, diseases of the circulatory system, diseases of the respiratory system, neoplasms, and diseases of the blood and blood-forming organs and certain disorders involving the immune mechanism ((E11, J00-J99, I00-I99, C00-D49, D5-D8) based on ICD-10 classification. ^{θ} Any medication prescribed as part of clinical trials or according to compassionate use or (e.g., hydroxychloroquine, azithromycin, remdesivir, tocilizumab, or sarilumab). ^{£} Assessed using ICD-10 codes for mood and anxiety disorders (F30-F48). 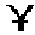 Assessed using ICD-10 codes (F00-F29 and F50-F99). ^{Ω} Included lithium or antiepileptic medications with mood stabilizing effects. ^{µ} Defined as having at least one of the following criteria: respiratory rate > 24 breaths/min or < 12 breaths/min, resting peripheral capillary oxygen saturation in ambient air < 90%, temperature > 40°C, or systolic blood pressure < 100 mm Hg. ^{K} Defined as having at least one of the following criteria: neutrophil-to-lymphocyte ratio higher than the median, *lymphocyte-to-C-reactive protein ratio* lower than the median, and plasma *lactate* levels *higher than 2 mmol*/*L.* * p-value is significant (p<0.05). | | | | | | |

**Table 10. Characteristics of patients receiving α2-antagonist antidepressants or not receiving any antidepressant in the matched and unmatched analytic samples.**

| | Exposed to any α2-antagonist antidepressant N=44 | Not exposed to any antidepressant N = 6,885 | Non-exposed matched group N = 44 | Exposed to any α2-antagonist antidepressant vs. Not exposed to any antidepressant | Exposed to any α2-antagonist antidepressant vs. Not exposed to any antidepressant | Exposed to any α2-antagonist antidepressant vs. Non-exposed matched group |
|---|---|---|---|---|---|---|
| | | | | Crude analysis | Analysis weighted by inverse-probability-weighting weights | Matched analytic sample analysis |
| | N (%) | N (%) | N (%) | Chi-square test (p-value) | Weighted Chi-square test (p-value) | Chi-square test (p-value) |
| *Characteristics* Age (years) | | | | 110.49 | 19.91 (<0.001*) | 3.96 (0.266) |
| *18-50* | 1 (2.3%) | 2,676 | 4 (9.1%) | | | |
| *51-70* | 4 (9.1%) | 2,410 | 8 (18.2%) | | | |
| *71-80* | 10 (22.7%) | 841 (12.2%) | 7 (15.9%) | | | |
| *81+* | 29 (65.9%) | 958 (13.9%) | 25 (56.8%) | 4.54 (0.033*) | 2.44 (0.118) | 0.19 (0.660) |
| Sex | | | | | | |
| *Women* | 29 (65.9%) | 3,350 | 26 (59.1%) | | | |
| *Men* | 15 (34.1%) | 3,535 | 18 (40.9%) | | | |
| Obesity ^{α} | | | | <0.01 (0.999) | 0.08 (0.779) | <0.01 (>0.99) |
| *Yes* | 6 (13.6%) | 868 (12.6%) | 5 (11.4%) | | | |
| *No* | 38 (86.4%) | 6,017 | 39 (88.6%) | | | |
| Smoking | | | | <0.01 (0.999) | 0.04 (0.840) | <0.01 (>0.99) |
| *Yes* | 4 (9.1%) | 560 (8.1%) | 4 (9.1%) | | | |
| *No* | 40 (90.9%) | 6,325 | 40 (90.9%) | | | |
| Any medical condition ^{β} | | | | 718.56 (<0.001*) | 0.13 (0.719) | <0.01 (>0.99) |
| *Yes* | 18 (40.9%) | 2,317 | 18 (40.9%) | | | |
| *No* | 26 (59.1%) | 4,568 | 26 (59.1%) | | | |
| Medication according to | | | | | | |
| compassionate use or as part of a clinical trial ^{θ} | | | | <0.01 (0.999) | 0.08 (0.780) | <0.01 (>0.99) |
| *Yes* | 7 (15.9%) | 1,139 | 6 (13.6%) | | | |
| *No* | 37 (84.1%) | 5,746 | 38 (86.4%) | | | |
| Any current mood or anxiety disorder ^{£} | | | | 36.56 (<0.001*) | 26.72 (>0.001*) | <0.01 (>0.99) |
| *Yes* | 5 (11.4%) | 76 (1.1%) | 5 (11.4%) | | | |
| *No* | 39 (88.6%) | 6,809 | 39 (88.6%) | | | |
| Any other current psychiatric disorder 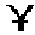 | | | | 0.67 (0.412) | 0.14 (0.711) | <0.01 (>0.99) |
| *Yes* | 3 (6.82%) | 235 (3.4%) | 3 (6.8%) | | | |
| *No* | 41 (93.2%) | 6,650 | 41 (93.2%) | | | |
| Any benzodiazepine or Z-drug | | | | 214.93 (<0.001*) | 63.13 (<0.001*) | 0.20 (0.655) |
| *Yes* | 30 (68.2%) | 513 (7.5%) | 27 (61.4%) | | | |
| *No* | 14 (31.8%) | 6,372 (92.5%) | 17 (38.6%) | | | |
| Any mood stabilizer medication ^{Ω} | | | | 140.37 (<0.001*) | 72.18 (<0.001*) | <0.01 (>0.99) |
| *Yes* | 15 (34.1%) | 188 (2.7%) | 15 (34.1%) | | | |
| *No* | 29 (65.9%) | 6,697 (97.3%) | 29 (65.9%) | | | |
| Any antipsychotic medication | | | | 112.17 (<0.001*) | 57.60 (<0.001*) | <0.01 (>0.99) |
| *Yes* | 13 (29.5%) | 173 (2.5%) | 13 (29.5%) | | | |
| *No* | 31 (70.5%) | 6,712 (97.5%) | 31 (70.5%) | | | |
| Clinical severity of Covid-19 at admission ^{µ} | | | | 5.20 (0.074) | 1.16 (0.561) | 0.83 (0.659) |
| *Yes* | 11 (25.0%) | 1,415 (20.6%) | 12 (27.3%) | | | |
| *No* | 16 (36.4%) | 1,673 (24.3%) | 19 (43.2%) | | | |
| *Missing* | 17 (38.6%) | 3,797 (55.1%) | 13 (29.5%) | | | |
| Biological severity of Covid-19 at admission ^{K} | | | | 9.39 (0.009*) | 1.16 (0.559) | 0.06 (0.971) |
| *Yes* | 19 (43.2%) | 2,213 (32.1%) | 20 (45.5%) | | | |
| *No* | 16 (36.4%) | 1,693 (24.6%) | 15 (34.1%) | | | |
| *Missing* | 9 (20.5%) | 2,979 (43.3%) | 9 (20.5%) | | | |

| | | | | | | |
|---|---|---|---|---|---|---|
| ^{α} Defined as having a body-mass index higher than 30 kg/m² or an International Statistical Classification of Diseases and Related Health Problems (ICD-10) diagnosis code for obesity (E66.0, E66.1, E66.2, E66.8, E66.9). ^{β} Assessed using ICD-10 codes for diabetes mellitus, diseases of the circulatory system, diseases of the respiratory system, neoplasms, and diseases of the blood and blood-forming organs and certain disorders involving the immune mechanism ((E11, J00-J99, I00-I99, C00-D49, D5-D8) based on ICD-10 classification. ^{θ} Any medication prescribed as part of clinical trials or according to compassionate use or (e.g., hydroxychloroquine, azithromycin, remdesivir, tocilizumab, or sarilumab). ^{£} Assessed using ICD-10 codes for mood and anxiety disorders (F30-F48). 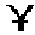 Assessed using ICD-10 codes (F00-F29 and F50-F99). ^{Ω} Included lithium or antiepileptic medications with mood stabilizing effects. ^{µ} Defined as having at least one of the following criteria: respiratory rate > 24 breaths/min or < 12 breaths/min, resting peripheral capillary oxygen saturation in ambient air < 90%, temperature > 40°C, or systolic blood pressure < 100 mm Hg. ^{K} Defined as having at least one of the following criteria: neutrophil-to-lymphocyte ratio higher than the median, *lymphocyte-to-C-reactive protein ratio* lower than the median, and plasma *lactate* levels *higher than 2 mmol*/*L.* * p-value is significant (p<0.05). | | | | | | |

**Table 11. Characteristics of patients receiving Escitalopram or not receiving any antidepressant in the matched and unmatched analytic samples.**

| | Exposed to Escitalopram N=84 | Not exposed to any antidepressant N = 6,885 | Non-exposed matched group N = 168 | Exposed to Escitalopram vs. Not exposed to any antidepressant | Exposed to Escitalopram vs. Not exposed to any antidepressant | Exposed to Escitalopram vs. Non-exposed matched group |
|---|---|---|---|---|---|---|
| | | | | Crude analysis | Analysis weighted by inverse-probability-weighting weights | Matched analytic sample analysis |
| | N (%) | N (%) | N (%) | Chi-square test (p-value) | Weighted Chi-square test (p-value) | Chi-square test (p-value) |
| *Characteristics* | | | | | | |
| Age (years) | | | | 128.80 | 26.59 (<0.001*) | 0.71 (0.871) |
| *18-50* | 4 (4.76%) | 2,676 (38.9%) | 8 (4.76%) | | | |
| *51-70* | 14 (16.7%) | 2,410 (35.0%) | 35 (20.8%) | | | |
| *71-80* | 23 (27.4%) | 841 (12.2%) | 41 (24.4%) | | | |
| 81+ | 43 (51.2%) | 958 (13.9%) | 84 (50.0%) | | | |
| Sex | | | | 10.06 (0.002*) | 3.71 (0.054) | 2.80 (0.094) |
| *Women* | 56 (66.7%) | 3,350 (48.7%) | 92 (54.8%) | | | |
| *Men* | 28 (33.3%) | 3,535 (51.3%) | 76 (45.2%) | | | |
| Obesity ^{α} | | | | 3.70 (0.054) | 0.55 (0.460) | 0.08 (0.775) |
| *Yes* | 17 (20.2%) | 868 (12.6%) | 30 (17.9%) | | | |
| *No* | 67 (79.8%) | 6,017 (87.4%) | 138 (82.1%) | | | |
| Smoking | | | | 2.10 (0.148) | 0.15 (0.702) | 0.42 (0.517) |
| *Yes* | 11 (13.1%) | 560 (8.1%) | 16 (9.52%) | | | |
| *No* | 73 (86.9%) | 6,325 (91.9%) | 152 (90.5%) | | | |
| Any medical condition ^{β} | | | | 13.82 (<0.001*) | 1.37 (0.242) | 1.08 (0.298) |
| *Yes* | 45 (53.6%) | 2,317 (33.7%) | 103 (61.3%) | | | |
| *No* | 39 (46.4%) | 4,568 (66.3%) | 65 (38.7%) | | | |
| Medication according to compassionate use or as part of a clinical trial ^{θ} | | | | 2.66 (0.103) | 0.48 (0.489) | 0.36 (0.548) |
| *Yes* | 20 (23.8%) | 1,139 (16.5%) | 33 (19.6%) | | | |
| *No* | 64 (76.2%) | 5,746 (83.5%) | 135 (80.4%) | | | |
| Any current mood or anxiety disorder ^{£} | | | | 55.89 (<0.001*) | 49.89 (<0.001*) | <0.01 (>0.99) |
| *Yes* | 9 (10.7%) | 76 (1.1%) | 18 (10.7%) | | | |
| *No* | 75 (89.3%) | 6,809 (98.9%) | 150 (89.3%) | | | |
| Any other current psychiatric disorder 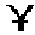 | | | | 71.92 (<0.001*) | 26.59 (<0.001*) | <0.01 (>0.99) |
| *Yes* | 18 (21.4%) | 235 (3.4%) | 36 (21.4%) | | | |
| *No* | 66 (78.6%) | 6,650 (96.6%) | 132 (78.6%) | | | |
| Any benzodiazepine or Z-drug | | | | 199.22 (<0.001*) | 67.38 (<0.001*) | <0.01 (>0.99) |
| *Yes* | 42 (50.0%) | 513 (7.5%) | 85 (50.6%) | | | |
| *No* | 42 (50.0%) | 6,372 (92.5%) | 83 (49.4%) | | | |
| Any mood stabilizer medication ^{Ω} | | | | 94.73 (<0.001*) | 34.12 (<0.001*) | 0.03 (0.868) |
| *Yes* | 18 (21.4%) | 188 (2.7%) | 33 (19.6%) | | | |
| *No* | 66 (78.6%) | 6,697 (97.3%) | 135 (80.4%) | | | |
| Any antipsychotic medication | | | | 48.81 (<0.001*) | 18.88 (<0.001*) | <0.01 (>0.99) |
| *Yes* | 13 (15.5%) | 173 (2.5%) | 26 (15.5%) | | | |
| *No* | 71 (84.5%) | 6,712 (97.5%) | 142 (84.5%) | | | |
| Clinical severity of Covid-19 at admission ^{µ} | | | | 12.67 (0.002*) | 0.65 (0.722) | 0.77 (0.680) |
| *Yes* | 25 (29.8%) | 1,415 (20.6%) | 56 (33.3%) | | | |
| *No* | 29 (34.5%) | 1,673 (24.3%) | 61 (36.3%) | | | |
| *Missing* | 30 (35.7%) | 3,797 (55.1%) | 51 (30.4%) | | | |
| Biological severity of Covid-19 at admission ^{K} | | | | 40.54 (<0.001*) | 6.84 (0.033*) | 1.21 (0.547) |
| *Yes* | 35 (41.7%) | 2,213 (32.1%) | 81 (48.2%) | | | |
| *No* | 40 (47.6%) | 1,693 (24.6%) | 68 (40.5%) | | | |
| *Missing* | 9 (10.7%) | 2,979 (43.3%) | 19 (11.3%) | | | |

| | | | | | | |
|---|---|---|---|---|---|---|
| ^{α} Defined as having a body-mass index higher than 30 kg/m² or an International Statistical Classification of Diseases and Related Health Problems (ICD-10) diagnosis code for obesity (E66.0, E66.1, E66.2, E66.8, E66.9). ^{β} Assessed using ICD-10 codes for diabetes mellitus, diseases of the circulatory system, diseases of the respiratory system, neoplasms, and diseases of the blood and blood-forming organs and certain disorders involving the immune mechanism ((E11, J00-J99, I00-I99, C00-D49, D5-D8) based on ICD-10 classification. ^{θ} Any medication prescribed as part of clinical trials or according to compassionate use or (e.g., hydroxychloroquine, azithromycin, remdesivir, tocilizumab, or sarilumab). ^{£} Assessed using ICD-10 codes for mood and anxiety disorders (F30-F48). 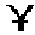 Assessed using ICD-10 codes (F00-F29 and F50-F99). ^{Ω} Included lithium or antiepileptic medications with mood stabilizing effects. ^{µ} Defined as having at least one of the following criteria: respiratory rate > 24 breaths/min or < 12 breaths/min, resting peripheral capillary oxygen saturation in ambient air < 90%, temperature > 40°C, or systolic blood pressure < 100 mm Hg. ^{K} Defined as having at least one of the following criteria: neutrophil-to-lymphocyte ratio higher than the median, *lymphocyte-to-C-reactive protein ratio* lower than the median, and plasma *lactate* levels *higher than 2 mmol*/*L.* * p-value is significant (p<0.05). | | | | | | |

**Table 12. Characteristics of patients receiving Fluoxetine or not receiving any antidepressant in the matched and unmatched analytic samples.**

| | Exposed to Fluoxetine N=35 | Not exposed to any antidepressant N = 6,885 | Non-exposed matched group N = 70 | Exposed to Fluoxetine vs. Not exposed to any antidepressant | Exposed to Fluoxetine vs. Not exposed to any antidepressant | Exposed to Fluoxetine vs. Non-exposed matched group |
|---|---|---|---|---|---|---|
| | | | | Crude analysis | Analysis weighted by inverse-probability-weighting weights | Matched analytic sample analysis |
| | N (%) | N (%) | N (%) | Chi-square test (p-value) | Weighted Chi-square test (p-value) | Chi-square test (p-value) |
| *Characteristics* Age (years) | | | | 13.70 (0.003*) | 0.78 (0.855) | 0.06 (0.996) |
| *18-50* | 4 (11.4%) | 2,676 (38.9%) | 9 (12.9%) | | | |
| *51-70* | 14 (40.0%) | 2,410 (35.0%) | 28 (40.0%) | | | |
| *71-80* | 8 (22.9%) | 841 (12.2%) | 16 (22.9%) | | | |
| 81+ | 9 (25.7%) | 958 (13.9%) | 17 (24.3%) | | | |
| Sex | | | | 2.27 (0.132) | 0.26 (0.613) | 1.72 (0.189) |
| *Women* | 22 (62.9%) | 3,350 (48.7%) | 33 (47.1%) | | | |
| *Men* | 13 (37.1%) | 3,535 (51.3%) | 37 (52.9%) | | | |
| Obesity ^{α} | | | | 16.72 (<0.001*) | 5.01 (0.025*) | 0.97 (0.325) |
| *Yes* | 13 (37.1%) | 868 (12.6%) | 18 (25.7%) | | | |
| *No* | 22 (62.9%) | 6,017 (87.4%) | 52 (74.3%) | | | |
| Smoking | | | | 2.66 (0.103*) | 0.48 (0.487) | 0.54 (0.463) |
| *Yes* | 6 (17.1%) | 560 (8.1%) | 7 (10.0%) | | | |
| *No* | 29 (82.9%) | 6,325 (91.9%) | 63 (90.0%) | | | |
| Any medical condition ^{β} | | | | 2.83 (0.092) | 0.12 (0.724) | 0.58 (0.445) |
| *Yes* | 17 (48.6%) | 2,317 (33.7%) | 41 (58.6%) | | | |
| *No* | 18 (51.4%) | 4,568 (66.3%) | 29 (41.4%) | | | |
| Medication according to compassionate use or as part of a clinical trial ^{θ} | | | | 0.10 (0.748) | 0.02 (0.903) | 0.08 (0.784) |
| *Yes* | 7 (20.0%) | 1,139 (16.5%) | 11 (15.7%) | | | |
| *No* | 28 (80.0%) | 5,746 (83.5%) | 59 (84.3%) | | | |
| Any current mood or anxiety disorder ^{£} | | | | 3.15 (0.076) | 0.01 (0.915) | <0.01 (>0.99) |
| *Yes* | 2 (5.7%) | 76 (1.1%) | 4 (5.7%) | | | |
| *No* | 33 (94.3%) | 6,809 (98.9%) | 66 (94.3%) | | | |
| Any other current psychiatric disorder 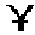 | | | | 0.08 (0.779) | 0.03 (0.854) | <0.01 (>0.99) |
| *Yes* | 2 (5.7%) | 235 (3.4%) | 4 (5.7%) | | | |
| *No* | 33 (94.3%) | 6,650 (96.6%) | 66 (94.3%) | | | |
| Any benzodiazepine or Z-drug | | | | 66.90 (<0.001*) | 12.33 (<0.001*) | <0.01 (>0.99) |
| *Yes* | 16 (45.7%) | 513 (7.5%) | 32 (45.7%) | | | |
| *No* | 19 (54.3%) | 6,372 (92.5%) | 38 (54.3%) | | | |
| Any mood stabilizer medication ^{Ω} | | | | 21.52 (<0.001*) | 0.31 (0.577) | <0.01 (>0.99) |
| *Yes* | 6 (17.1%) | 188 (2.7%) | 12 (17.1%) | | | |
| *No* | 29 (82.9%) | 6,697 (97.3%) | 58 (82.9%) | | | |
| Any antipsychotic medication | | | | 35.41 (<0.001*) | 16.92 (<0.001*) | <0.01 (>0.99) |
| *Yes* | 7 (20.0%) | 173 (2.5%) | 14 (20.0%) | | | |
| *No* | 28 (80.0%) | 6,712 (97.5%) | 56 (80.0%) | | | |
| Clinical severity of Covid-19 at admission ^{µ} | | | | 14.77 (0.001*) | 1.87 (0.393) | 0.32 (0.853) |
| *Yes* | 13 (37.1%) | 1,415 (20.6%) | 24 (34.3%) | | | |
| *No* | 14 (40.0%) | 1,673 (24.3%) | 32 (45.7%) | | | |
| *Missing* | 8 (22.9%) | 3,797 (55.1%) | 14 (20.0%) | | | |
| Biological severity of Covid-19 at admission ^{K} | | | | 12.13 (0.002*) | 1.36 (0.507) | 1.94 (0.380) |
| *Yes* | 16 (45.7%) | 2,213 (32.1%) | 40 (57.1%) | | | |
| *No* | 14 (40.0%) | 1,693 (24.6%) | 25 (35.7%) | | | |
| *Missing* | 5 (14.3%) | 2,979 (43.3%) | 5 (7.1%) | | | |

| | | | | | | |
|---|---|---|---|---|---|---|
| ^{α} Defined as having a body-mass index higher than 30 kg/m² or an International Statistical Classification of Diseases and Related Health Problems (ICD-10) diagnosis code for obesity (E66.0, E66.1, E66.2, E66.8, E66.9). ^{β} Assessed using ICD-10 codes for diabetes mellitus, diseases of the circulatory system, diseases of the respiratory system, neoplasms, and diseases of the blood and blood-forming organs and certain disorders involving the immune mechanism ((E11, J00-J99, I00-I99, C00-D49, D5-D8) based on ICD-10 classification. ^{θ} Any medication prescribed as part of clinical trials or according to compassionate use or (e.g., hydroxychloroquine, azithromycin, remdesivir, tocilizumab, or sarilumab). ^{£} Assessed using ICD-10 codes for mood and anxiety disorders (F30-F48). 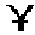 Assessed using ICD-10 codes (F00-F29 and F50-F99). ^{Ω} Included lithium or antiepileptic medications with mood stabilizing effects. ^{µ} Defined as having at least one of the following criteria: respiratory rate > 24 breaths/min or < 12 breaths/min, resting peripheral capillary oxygen saturation in ambient air < 90%, temperature > 40°C, or systolic blood pressure < 100 mm Hg. ^{K} Defined as having at least one of the following criteria: neutrophil-to-lymphocyte ratio higher than the median, *lymphocyte-to-C-reactive protein ratio* lower than the median, and plasma *lactate* levels *higher than 2 mmol*/*L.* * p-value is significant (p<0.05). | | | | | | |

**Table 13. Characteristics of patients receiving Paroxetine or not receiving any antidepressant in the matched and unmatched analytic samples.**

| | Exposed to Paroxetine N=79 | Not exposed to any antidepressant N = 6,885 | Non-exposed matched group N = 158 | Exposed to Paroxetine vs. Not exposed to any antidepressant | Exposed to Paroxetine vs. Not exposed to any antidepressant | Exposed to Paroxetine vs. Non-exposed matched group |
|---|---|---|---|---|---|---|
| | | | | Crude analysis | Analysis weighted by inverse-probability-weighting weights | Matched analytic sample analysis |
| | N (%) | N (%) | N (%) | Chi-square test (p-value) | Weighted Chi-square test (p-value) | Chi-square test (p-value) |
| *Characteristics* | | | | | | |
| Age (years) | | | | 52.47 (<0.001*) | 9.20 (0.027*) | 0.04 (0.998) |
| *18-50* | 7 (8.86%) | 2,676 (38.9%) | 14 (8.86%) | | | |
| *51-70* | 26 (32.9%) | 2,410 (35.0%) | 51 (32.3%) | | | |
| *71-80* | 17 (21.5%) | 841 (12.2%) | 33 (20.9%) | | | |
| *81+* | 29 (36.7%) | 958 (13.9%) | 60 (38.0%) | | | |
| Sex | | | | 1.84 (0.175) | 0.33 (0.568) | 0.76 (0.382) |
| *Women* | 45 (57.0%) | 3,350 (48.7%) | 79 (50.0%) | | | |
| *Men* | 34 (43.0%) | 3,535 (51.3%) | 79 (50.0%) | | | |
| Obesity ^{α} | | | | 4.82 (0.028*) | 0.78 (0.337) | 0.08 (0.773) |
| *Yes* | 17 (21.5%) | 868 (12.6%) | 30 (19.0%) | | | |
| *No* | 62 (78.5%) | 6,017 (87.4%) | 128 (81.0%) | | | |
| Smoking | | | | 8.27 (0.004*) | 1.07 (0.300) | <0.01 (0.951) |
| *Yes* | 14 (17.7%) | 560 (8.1%) | 26 (16.5%) | | | |
| *No* | 65 (82.3%) | 6,325 (91.9%) | 132 (83.5%) | | | |
| Any medical condition ^{β} | | | | 14.14 (<0.001*) | 1.85 (0.174) | 0.63 (0.428) |
| *Yes* | 43 (54.4%) | 2,317 (33.7%) | 96 (60.8%) | | | |
| *No* | 36 (45.6%) | 4,568 (66.3%) | 62 (39.2%) | | | |
| Medication according to compassionate use or as part of a clinical trial ^{θ} | | | | 1.06 (0.303) | 0.01 (0.916) | <0.01 (>0.99) |
| *Yes* | 17 (21.5%) | 1,139 (16.5%) | 35 (22.2%) | | | |
| *No* | 62 (78.5%) | 5,746 (83.5%) | 123 (77.8%) | | | |
| Any current mood or anxiety disorder ^{£} | | | | 45.05 (<0.001*) | 30.55 (<0.001*) | <0.01 (>0.99) |
| *Yes* | 8 (10.1%) | 76 (1.1%) | 16 (10.1%) | | | |
| *No* | 71 (89.9%) | 6,809 (98.9%) | 142 (89.9%) | | | |
| Any other current psychiatric disorder 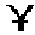 | | | | 42.32 (<0.001*) | 15.22 (<0.001*) | <0.01 (>0.99) |
| *Yes* | 14 (17.7%) | 235 (3.4%) | 28 (17.7%) | | | |
| *No* | 65 (82.3%) | 6,650 (96.6%) | 130 (82.3%) | | | |
| Any benzodiazepine or Z-drug | | | | 228.29 (<0.001*) | 66.03 (<0.001*) | 0.17 (0.679) |
| *Yes* | 43 (54.4%) | 513 (7.5%) | 80 (50.6%) | | | |
| *No* | 36 (45.6%) | 6,372 (92.5%) | 78 (49.4%) | | | |
| Any mood stabilizer medication ^{Ω} | | | | 47.71 (<0.001*) | 30.02 (<0.001*) | <0.01 (>0.99) |
| *Yes* | 13 (16.5%) | 188 (2.7%) | 26 (16.5%) | | | |
| *No* | 66 (83.5%) | 6,697 (97.3%) | 132 (83.5%) | | | |
| Any antipsychotic medication | | | | 99.27 (<0.001*) | 34.87 (<0.001*) | <0.01 (>0.99) |
| *Yes* | 17 (21.5%) | 173 (2.5%) | 34 (21.5%) | | | |
| *No* | 62 (78.5%) | 6,712 (97.5%) | 124 (78.5%) | | | |
| Clinical severity of Covid-19 at admission ^{µ} | | | | 24.58 (<0.001*) | 4.17 (0.125) | 0.75 (0.688) |
| *Yes* | 23 (29.1%) | 1,415 (20.6%) | 49 (31.0%) | | | |
| *No* | 34 (43.0%) | 1,673 (24.3%) | 59 (37.3%) | | | |
| *Missing* | 22 (27.8%) | 3,797 (55.1%) | 50 (31.6%) | | | |
| Biological severity of Covid-19 at admission ^{K} | | | | 33.15 (<0.001*) | 4.43 (0.109) | 0.96 (0.618) |
| *Yes* | 39 (49.4%) | 2,213 (32.1%) | 82 (51.9%) | | | |
| *No* | 32 (40.5%) | 1,693 (24.6%) | 55 (34.8%) | | | |
| *Missing* | 8 (10.1%) | 2,979 (43.3%) | 21 (13.3%) | | | |

| | | | | | | |
|---|---|---|---|---|---|---|
| ^{α} Defined as having a body-mass index higher than 30 kg/m² or an International Statistical Classification of Diseases and Related Health Problems (ICD-10) diagnosis code for obesity (E66.0, E66.1, E66.2, E66.8, E66.9). ^{β} Assessed using ICD-10 codes for diabetes mellitus, diseases of the circulatory system, diseases of the respiratory system, neoplasms, and diseases of the blood and blood-forming organs and certain disorders involving the immune mechanism ((E11, J00-J99, I00-I99, C00-D49, D5-D8) based on ICD-10 classification. ^{θ} Any medication prescribed as part of clinical trials or according to compassionate use or (e.g., hydroxychloroquine, azithromycin, remdesivir, tocilizumab, or sarilumab). ^{£} Assessed using ICD-10 codes for mood and anxiety disorders (F30-F48). 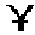 Assessed using ICD-10 codes (F00-F29 and F50-F99). ^{Ω} Included lithium or antiepileptic medications with mood stabilizing effects. ^{µ} Defined as having at least one of the following criteria: respiratory rate > 24 breaths/min or < 12 breaths/min, resting peripheral capillary oxygen saturation in ambient air < 90%, temperature > 40°C, or systolic blood pressure < 100 mm Hg. ^{K} Defined as having at least one of the following criteria: neutrophil-to-lymphocyte ratio higher than the median, *lymphocyte-to-C-reactive protein ratio* lower than the median, and plasma *lactate* levels *higher than 2 mmol*/*L.* * p-value is significant (p<0.05). | | | | | | |

**Table 14. Characteristics of patients receiving Venlafaxine or not receiving any antidepressant in the matched and unmatched analytic samples.**

| | Exposed to Venlafaxine N=49 | Not exposed to any antidepressant N = 6,885 | Non-exposed matched group N = 98 | Exposed to Venlafaxine vs. Not exposed to any antidepressant | Exposed to Venlafaxine vs. Not exposed to any antidepressant | Exposed to Venlafaxine vs. Non-exposed matched group |
|---|---|---|---|---|---|---|
| | | | | Crude analysis | Analysis weighted by inverse-probability-weighting weights | Matched analytic sample analysis |
| | N (%) | N (%) | N (%) | Chi-square test (p-value) | Weighted Chi-square test (p-value) | Chi-square test (p-value) |
| *Characteristics* | | | | | | |
| Age (years) | | | | 40.73 (<0.001*) | 6.41 (0.093) | 1.89 (0.595) |
| *18-50* | 2 (4.08%) | 2,676 (38.9%) | 10 (10.2%) | | | |
| *51-70* | 17 (34.7%) | 2,410 (35.0%) | 31 (31.6%) | | | |
| *71-80* | 11 (22.4%) | 841 (12.2%) | 24 (24.5%) | | | |
| 81+ | 19 (38.8%) | 958 (13.9%) | 33 (33.7%) | | | |
| Sex | | | | 1.76 (0.185) | 2.46 (0.117) | 2.48 (0.115) |
| *Women* | 29 (59.2%) | 3,350 (48.7%) | 43 (43.9%) | | | |
| *Men* | 20 (40.8%) | 3,535 (51.3%) | 55 (56.1%) | | | |
| Obesity ^{α} | | | | 5.17 (0.023*) | 0.97 (0.324) | 0.42 (0.515) |
| *Yes* | 12 (24.5%) | 868 (12.6%) | 18 (18.4%) | | | |
| *No* | 37 (75.5%) | 6,017 (87.4%) | 80 (81.6%) | | | |
| Smoking | | | | 11.37 (0.001*) | 1.66 (0.198) | <0.01 (>0.99) |
| *Yes* | 11 (22.4%) | 560 (8.1%) | 22 (22.4%) | | | |
| *No* | 38 (77.6%) | 6,325 (91.9%) | 76 (77.6%) | | | |
| Any medical condition ^{β} | | | | 15.31 (<0.001*) | 2.28 (0.131) | 0.46 (0.498) |
| *Yes* | 30 (61.2%) | 2,317 (33.7%) | 67 (68.4%) | | | |
| *No* | 19 (38.8%) | 4,568 (66.3%) | 31 (31.6%) | | | |
| Medication according to compassionate use or as part of a clinical trial ^{θ} | | | | 0.02 (0.881) | 0.01 (0.926) | 0.05 (0.828) |
| *Yes* | 9 (18.4%) | 1,139 (16.5%) | 21 (21.4%) | | | |
| *No* | 40 (81.6%) | 5,746 (83.5%) | 77 (78.6%) | | | |
| Any current mood or anxiety disorder ^{£} | | | | 162.11 (<0.001*) | 104.86 (<0.001*) | <0.01 (>0.99) |
| *Yes* | 11 (22.4%) | 76 (1.1%) | 22 (22.4%) | | | |
| *No* | 38 (77.6%) | 6,809 (98.9%) | 76 (77.6%) | | | |
| Any other current psychiatric disorder 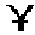 | | | | 14.00 (<0.001*) | 0.78 (0.376) | <0.01 (>0.99) |
| *Yes* | 7 (14.3%) | 235 (3.4%) | 14 (14.3%) | | | |
| *No* | 42 (85.7%) | 6,650 (96.6%) | 84 (85.7%) | | | |
| Any benzodiazepine or Z-drug | | | | 201.99 (<0.001*) | 48.88 (<0.001*) | 0.17 (0.677) |
| *Yes* | 31 (63.3%) | 513 (7.5%) | 57 (58.2%) | | | |
| *No* | 18 (36.7%) | 6,372 (92.5%) | 41 (41.8%) | | | |
| Any mood stabilizer medication ^{Ω} | | | | 123.46 (<0.001*) | 84.45 (<0.001*) | 0.04 (0.846) |
| *Yes* | 15 (30.6%) | 188 (2.7%) | 27 (27.6%) | | | |
| *No* | 34 (69.4%) | 6,697 (97.3%) | 71 (72.4%) | | | |
| Any antipsychotic medication | | | | 82.23 (<0.001*) | 50.18 (<0.001*) | <0.01 (>0.99) |
| *Yes* | 12 (24.5%) | 173 (2.5%) | 24 (24.5%) | | | |
| *No* | 37 (75.5%) | 6,712 (97.5%) | 74 (75.5%) | | | |
| Clinical severity of Covid-19 at admission ^{µ} | | | | 13.88 (0.001*) | 3.05 (0.218) | 0.16 (0.921) |
| *Yes* | 16 (32.7%) | 1,415 (20.6%) | 34 (34.7%) | | | |
| *No* | 19 (38.8%) | 1,673 (24.3%) | 39 (39.8%) | | | |
| *Missing* | 14 (28.6%) | 3,797 (55.1%) | 25 (25.5%) | | | |
| Biological severity of Covid-19 at admission ^{K} | | | | 22.31 (<0.001*) | 3.6 (0.165) | 0.30 (0.862) |
| *Yes* | 27 (55.1%) | 2,213 (32.1%) | 53 (54.1%) | | | |
| *No* | 17 (34.7%) | 1,693 (24.6%) | 32 (32.7%) | | | |
| *Missing* | 5 (10.2%) | 2,979 (43.3%) | 13 (13.3%) | | | |

| | | | | | | |
|---|---|---|---|---|---|---|
| ^{α} Defined as having a body-mass index higher than 30 kg/m² or an International Statistical Classification of Diseases and Related Health Problems (ICD-10) diagnosis code for obesity (E66.0, E66.1, E66.2, E66.8, E66.9). ^{β} Assessed using ICD-10 codes for diabetes mellitus, diseases of the circulatory system, diseases of the respiratory system, neoplasms, and diseases of the blood and blood-forming organs and certain disorders involving the immune mechanism ((E11, J00-J99, I00-I99, C00-D49, D5-D8) based on ICD-10 classification. ^{θ} Any medication prescribed as part of clinical trials or according to compassionate use or (e.g., hydroxychloroquine, azithromycin, remdesivir, tocilizumab, or sarilumab). ^{£} Assessed using ICD-10 codes for mood and anxiety disorders (F30-F48). 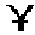 Assessed using ICD-10 codes (F00-F29 and F50-F99). ^{Ω} Included lithium or antiepileptic medications with mood stabilizing effects. ^{µ} Defined as having at least one of the following criteria: respiratory rate > 24 breaths/min or < 12 breaths/min, resting peripheral capillary oxygen saturation in ambient air < 90%, temperature > 40°C, or systolic blood pressure < 100 mm Hg. ^{K} Defined as having at least one of the following criteria: neutrophil-to-lymphocyte ratio higher than the median, *lymphocyte-to-C-reactive protein ratio* lower than the median, and plasma *lactate* levels *higher than 2 mmol*/*L.* * p-value is significant (p<0.05). | | | | | | |

**Table 15. Characteristics of patients receiving Mirtazapine or not receiving any antidepressant in the matched and unmatched analytic samples.**

| | Exposed to Mirtazapine N=44 | Not exposed to any antidepressant N = 6,885 | Non-exposed matched group N = 88 | Exposed to Mirtazapine vs. Not exposed to any antidepressant | Exposed to Mirtazapine vs. Not exposed to any antidepressant | Exposed to Mirtazapine vs. Non-exposed matched group |
|---|---|---|---|---|---|---|
| | | | | Crude analysis | Analysis weighted by inverse-probability-weighting weights | Matched analytic sample analysis |
| | N (%) | N (%) | N (%) | Chi-square test (p-value) | Weighted Chi-square test (p-value) | Chi-square test (p-value) |
| *Characteristics* | | | | | | |
| Age (years) | | | | 110.49 (<0.001*) | 19.91 (<0.001*) | 2.96 (0.397) |
| *18-50* | 1 (2.3%) | 2,676 (38.9%) | 5 (5.7%) | | | |
| *51-70* | 4 (9.1%) | 2,410 (35.0%) | 16 (18.2%) | | | |
| *71-80* | 10 (22.7%) | 841 (12.2%) | 16 (18.2%) | | | |
| *81+* | 29 (65.9%) | 958 (13.9%) | 51 (58.0%) | | | |
| Sex | | | | 4.54 (0.033*) | 2.44 (0.118) | 1.40 (0.237) |
| *Women* | 29 (65.9%) | 3,350 (48.7%) | 47 (53.4%) | | | |
| *Men* | 15 (34.1%) | 3,535 (51.3%) | 41 (46.6%) | | | |
| Obesity ^{α} | | | | <0.01 (>0.99) | 0.08 (0.779) | <0.01 (>0.99) |
| *Yes* | 6 (13.6%) | 868 (12.6%) | 13 (14.8%) | | | |
| *No* | 38 (86.4%) | 6,017 (87.4%) | 75 (85.2%) | | | |
| Smoking | | | | <0.01 (>0.99) | 0.04 (0.840) | <0.01 (>0.99) |
| *Yes* | 4 (9.1%) | 560 (8.1%) | 7 (8.0%) | | | |
| *No* | 40 (90.9%) | 6,325 (91.9%) | 81 (92.0%) | | | |
| Any medical condition ^{β} | | | | 0.73 (0.393) | 0.13 (0.719) | 0.03 (0.852) |
| *Yes* | 18 (40.9%) | 2,317 (33.7%) | 39 (44.3%) | | | |
| *No* | 26 (59.1%) | 4,568 (66.3%) | 49 (55.7%) | | | |
| Medication according to compassionate use or as part of a clinical trial ^{θ} | | | | <0.01 (>0.99) | 0.08 (0.780) | 0.06 (0.811) |
| *Yes* | 7 (15.9%) | 1,139 (16.5%) | 17 (19.3%) | | | |
| *No* | 37 (84.1%) | 5,746 (83.5%) | 71 (80.7%) | | | |
| Any current mood or anxiety disorder ^{£} | | | | 31.45 (<0.001*) | 26.72 (<0.001*) | <0.01 (>0.99) |
| *Yes* | 5 (11.4%) | 76 (1.1%) | 10 (11.4%) | | | |
| *No* | 39 (88.6%) | 6,809 (98.9%) | 78 (88.6%) | | | |
| Any other current psychiatric disorder 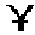 | | | | 0.67 (0.412) | 0.14 (0.711) | <0.01 (>0.99) |
| *Yes* | 3 (6.8%) | 235 (3.4%) | 6 (6.8%) | | | |
| *No* | 41 (93.2%) | 6,650 (96.6%) | 82 (93.2%) | | | |
| Any benzodiazepine or Z-drug | | | | 214.93 (<0.001*) | 63.13 (<0.001*) | 0.10 (0.747) |
| *Yes* | 30 (68.2%) | 513 (7.5%) | 56 (63.6%) | | | |
| *No* | 14 (31.8%) | 6,372 (92.5%) | 32 (36.4%) | | | |
| Any mood stabilizer medication ^{Ω} | | | | 140.37 (<0.001*) | 72.18 (<0.001*) | <0.01 (>0.99) |
| *Yes* | 15 (34.1%) | 188 (2.7%) | 29 (33.0%) | | | |
| *No* | 29 (65.9%) | 6,697 (97.3%) | 59 (67.0%) | | | |
| Any antipsychotic medication | | | | 112.17 (<0.001*) | 57.60 (<0.001*) | <0.01 (>0.99) |
| *Yes* | 13 (29.5%) | 173 (2.5%) | 25 (28.4%) | | | |
| *No* | 31 (70.5%) | 6,712 (97.5%) | 63 (71.6%) | | | |
| Clinical severity of Covid-19 at admission ^{µ} | | | | 5.20 (0.074) | 1.16 (0.561) | 1.25 (0.536) |
| *Yes* | 11 (25.0%) | 1,415 (20.6%) | 28 (31.8%) | | | |
| *No* | 16 (36.4%) | 1,673 (24.3%) | 34 (38.6%) | | | |
| *Missing* | 17 (38.6%) | 3,797 (55.1%) | 26 (29.5%) | | | |
| Biological severity of Covid-19 at admission ^{K} | | | | 9.39 (0.009*) | 1.16 (0.559) | 0.56 (0.756) |
| *Yes* | | | | 19 (43.2%) | 2,213 (32.1%) | 43 (48.9%) |
| *No* | | | | 16 (36.4%) | 1,693 (24.6%) | 31 (35.2%) |
| *Missing* | | | | 9 (20.5%) | 2,979 (43.3%) | 14 (15.9%) |

| | | | | | | |
|---|---|---|---|---|---|---|
| ^{α} Defined as having a body-mass index higher than 30 kg/m² or an International Statistical Classification of Diseases and Related Health Problems (ICD-10) diagnosis code for obesity (E66.0, E66.1, E66.2, E66.8, E66.9). ^{β} Assessed using ICD-10 codes for diabetes mellitus, diseases of the circulatory system, diseases of the respiratory system, neoplasms, and diseases of the blood and blood-forming organs and certain disorders involving the immune mechanism ((E11, J00-J99, I00-I99, C00-D49, D5-D8) based on ICD-10 classification. ^{θ} Any medication prescribed as part of clinical trials or according to compassionate use or (e.g., hydroxychloroquine, azithromycin, remdesivir, tocilizumab, or sarilumab). ^{£} Assessed using ICD-10 codes for mood and anxiety disorders (F30-F48). 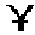 Assessed using ICD-10 codes (F00-F29 and F50-F99). ^{Ω} Included lithium or antiepileptic medications with mood stabilizing effects. ^{µ} Defined as having at least one of the following criteria: respiratory rate > 24 breaths/min or < 12 breaths/min, resting peripheral capillary oxygen saturation in ambient air < 90%, temperature > 40°C, or systolic blood pressure < 100 mm Hg. ^{K} Defined as having at least one of the following criteria: neutrophil-to-lymphocyte ratio higher than the median, *lymphocyte-to-C-reactive protein ratio* lower than the median, and plasma *lactate* levels *higher than 2 mmol*/*L.* * p-value is significant (p<0.05). | | | | | | |

**Table 16. Effect of baseline characteristics on the association between exposure to any antidepressant and the composite endpoint of death or intubation in the cohort of adult inpatients with Covid-19 (N=7,345).**

| | Multivariable Cox Regression models including successively all characteristics and the corresponding interaction term (characteristic*antidepressant exposure) | |
|---|---|---|
| | HR (SE) | p-value |
| *Characteristics* Age (years) | | |
| *18-50* | Ref. | |
| *51-70* | 0.67 (0.50) | 0.415 |
| *71-80* | 0.51 (0.49) | 0.164 |
| *81+* | 0.64 (0.47) | 0.338 |

| Sex | | |
|---|---|---|
| *Women* | Ref. | |
| *Men* | 0.86 (0.18) | 0.412 |

| Obesity ^{α} | | |
|---|---|---|
| *Yes* | 0.93 (0.22) | 0.723 |
| *No* | Ref. | |

| Smoking | | |
|---|---|---|
| *Yes* | 1.01 (0.23) | 0.977 |
| *No* | Ref. | |

| Any medical condition ^{β} | | |
|---|---|---|
| *Yes* | 1.36 (0.22) | 0.158 |
| *No* | Ref. | |

| Medication according to compassionate use or as part of a clinical trial ^{θ} | | |
|---|---|---|
| *Yes* | 0.72 (0.24) | 0.166 |
| *No* | Ref. | |

| Any current mood or anxiety disorder ^{£} | | |
|---|---|---|
| *Yes* | 0.62 (0.27) | 0.075 |
| *No* | Ref. | |

| Any other current psychiatric disorder 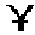 | | |
|---|---|---|
| *Yes* | 0.89 (0.21) | 0.581 |
| *No* | Ref. | |

| Any benzodiazepine or Z-drug | | |
|---|---|---|
| *Yes* | 0.70 (0.18) | 0.058 |
| *No* | Ref. | |

| Any mood stabilizer medication ^{Ω} | | |
|---|---|---|
| *Yes* | 0.71 (0.29) | 0.220 |
| *No* | Ref. | |

| Any antipsychotic medication | | |
|---|---|---|
| *Yes* | 0.90 (0.25) | 0.684 |
| *No* | Ref. | |

| Clinical severity of Covid-19 at admission ^{µ} | | |
|---|---|---|
| *Yes* | 0.77 (0.21) | 0.220 |
| *No* | Ref. | |
| *Missing* | 1.33 (0.23) | 0.201 |

| Biological severity of Covid-19 at admission ^{K} | | |
|---|---|---|
| *Yes* | 0.93 (0.21) | 0.739 |
| *No* | Ref. | |
| *Missing* | 2.30 (0.29) | 0.004* |

| | | |
|---|---|---|
| ^{α} Defined as having a body-mass index higher than 30 kg/m² or an International Statistical Classification of Diseases and Related Health Problems (ICD-10) diagnosis code for obesity (E66.0, E66.1, E66.2, E66.8, E66.9). ^{β} Assessed using ICD-10 codes for diabetes mellitus, diseases of the circulatory system, diseases of the respiratory system, neoplasms, and diseases of the blood and blood-forming organs and certain disorders involving the immune mechanism ((E11, J00-J99, I00-I99, C00-D49, D5-D8) based on ICD-10 classification. ^{θ} Any medication prescribed as part of clinical trials or according to compassionate use or (e.g., hydroxychloroquine, azithromycin, remdesivir, tocilizumab, or sarilumab). ^{£} Assessed using ICD-10 codes for mood and anxiety disorders (F30-F48). 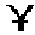 Assessed using ICD-10 codes (F00-F29 and F50-F99). ^{Ω} Included lithium or antiepileptic medications with mood stabilizing effects. ^{µ} Defined as having at least one of the following criteria: respiratory rate > 24 breaths/min or < 12 breaths/min, resting peripheral capillary oxygen saturation in ambient air < 90%, temperature > 40°C, or systolic blood pressure < 100 mm Hg. ^{K} Defined as having at least one of the following criteria: neutrophil-to-lymphocyte ratio higher than the median, *lymphocyte-to-C-reactive protein ratio* lower than the median, and plasma *lactate* levels *higher than 2 mmol*/*L.* * p-value is significant (p<0.05). | | |

**Table 17. Associations between antidepressant dosage and the primary endpoint.** 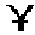

| | **Full sample** | **Multivariable (Weighted) Cox regression analysis** | **Analysis weighted by inverse-probability-weighting weights** | **Matched analytic sample** | **Univariate Cox regression in a matched analytic sample 1:2** |
|---|---|---|---|---|---|
| | Number of events / of patients | Number HR (CI 95%; p-value) | HR (CI 95%; p-value) | Number of events / Number of patients (N/%) | HR (CI 95%; p-value) |
| ***Any antidepressant (n=381)*** | | | | | |
| *Low dosage* | 54/198 (27.3%) | Ref. | Ref. | 54/198 (27.3%) | Ref. |
| *High dosage* | 59/183 (32.2%) | 1.31 (0.91-1.90; 0.140) | 1.21 (0.78 -1.87; 0.388) | 59/183 (32.2%) | 1.27 (0.88 - 1.83; 0.209) 1.92 - |
| *No antidepressant* | 1,188 / 6,885 (17.3%) | 1.78 (1.33 - 2.37; <0.001*) | 1.74 (1.26 - 2.39; <0.001*) | 194/460 (42.2%) | (0.88 1.83; <0.001*) |

| ***SSRIs (n=213)*** | | | | | |
|---|---|---|---|---|---|
| *Low dosage* | 31/129 (24.0%) | Ref. | Ref. | 31/129 (24.0%) | Ref. |
| *High dosage* | 29/84 (34.5%) | 1.51 (0.93 - 2.45; 0.094) | 1.54 (0.84 - 2.81; 0.164) | 29/84 (34.5%) | 1.64 (0.99 - 2.72; 0.056) 2.46 (1.66 - 3.66; |
| *No antidepressant* | 1,188 / 6,885 (17.3%) | 2.12 (1.53 - 2.97; <0.001*) | 2.16 (1.42 - 3.31; <0.001*) | 117/257 (45.5%) | <0.001*) |

| ***SNRIs (n=55)*** | | | | | |
|---|---|---|---|---|---|
| *Low dosage* | 6/32 (18.8%) | Ref. | Ref. | 6/32 (18.8%) | Ref. |
| *High dosage* | 6/23 (13.0%) | 1.41 (0.48 - 4.15; 0.534) | 1.23 (0.34 - 4.43; 0.746) | 6/23 (13.0%) | 1.49 (0.48 - 4.62; 0.494) |
| *No antidepressant* | 1,188 / 6,885 (17.3%) | 2.35 (1.07 - 5.17; 0.034*) | 2.24 (0.93 - 5.39; 0.071) | 27/71 (38.0%) | 2.42 (1.00 - 5.86; 0.051) |

| ***Tricyclic antidepressants (n=50)*** | | | | | |
|---|---|---|---|---|---|
| *Low dosage* | 10 / 26 (38.5%) | Ref. | Ref. | 10/26 (38.5%) | Ref. |
| *High dosage* | 6/14 (42.9%) | 1.08 (0.39 - 2.99; 0.885) | 0.83 (0.28 - 2.51; 0.744) | 6/14 (42.9%) | 1.01 (0.36 - 2.80; 0.984) |
| *No antidepressant* | 1,188 / 6,885 (17.3%) | 1.20 (0.63 - 2.28; 0.576) | 0.96 (0.52 -1.79; 0.900) | 20/59 (33.9%) | 1.18 (0.55 - 2.53; 0.668) |

| ***Tetracyclic antidepressants (n=87)*** | | | | | |
|---|---|---|---|---|---|
| *Low dosage* | 30/72 (41.7%) | Ref. | Ref. | 30/72 (41.7%) | Ref. |
| *High dosage* | 10 / 15 (66.7%) | 2.21 (1.08 - 4.56; 0.031*) | 1.92 (0.88 - 4.20; 0.102) | 10/15 (66.7%) | 2.04 (1.00 - 4.19; 0.051) |
| *No antidepressant* | 1,188 / 6,885 (17.3%) | 1.35 (0.93 -1.97; 0.113) | 1.25 (0.82 - 1.89; 0.302) | 41/94 (45.1%) | 1.20 (0.75 -1.93; 0.443) |

| ***Presynaptic α2-antagonist antidepressant (n=41)*** | | | | | |
|---|---|---|---|---|---|
| *Low dosage* | 5/26 (19.2%) | Ref. | Ref. | 5/26 (19.2%) | Ref. |
| *High dosage* | 4/15 (26.7%) | 3.21 (0.82 -12.51; 0.093) | 1.79 (0.45 - 7.22; 0.410) | 4/15 (26.7%) | 1.31 (0.35 - 4.88; 0.692) |
| *No antidepressant* | 1,188 / 6,885 (17.3%) | 3.62 (1.32 - 9.94; 0.013*) | 2.96 (1.08 - 8.12; 0.035*) | 16/44 (36.4%) | 2.15 (0.79 - 5.88; 0.136) |

| | | | | | |
|---|---|---|---|---|---|
| 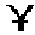 Information was lacking to confidently assess antidepressant dosages in 79 participants (17.2%), who were excluded from these analyses. * p-value is significant (p<0.05). | | | | | |

**Table 18. Associations between exposure to any antidepressant or to SSRIs and the composite endpoint of death or intubation among patients admitted to ICU (N = 593).**

| | **Full sample** | **Multivariable Cox regression analysis** | **Analysis weighted by inverse-probability-weighting weights** | **Matched analytic sample** | **Univariate Cox regression in a matched analytic sample** |
|---|---|---|---|---|---|
| | **Number of events** / **Number of patients (N / %)** | **HR (CI 95%; p-value)** | **HR (CI 95%; p-value)** | **Number of events / Number of patients (N / %)** | **HR (CI 95%; p-value)** |
| ***No antidepressant (n=565)*** | 513 / 565 (90.8%) | Ref. | Ref. | Ref. | Ref. |
| ***Any antidepressant (n=28)*** | 25 / 28 (89.3%) | 0.91 (0.47 - 1.10; 0.123) | 0.63 (0.40 - 0.99; 0.044*) | 54 / 56 (96.4%) | 0.89 (0.55 - 1.43; 0.628) |
| ***SSRIs (n=14)*** | 12 / 14 (35.7%) | 0.85 (0.47 - 1.52; 0.579) | 0.82 (0.42 - 1.58; 0.547) | 27 / 28 (96.4%) | 1.00 (0.50 - 2.00; 0.996) |

| | | | | | |
|---|---|---|---|---|---|
| * p-value is significant (p<0.05). | | | | | |

**Table 19. Associations between exposure to any antidepressant or to SSRIs in the past 3 months before hospital admission and not during the hospitalization, and the composite endpoint of death or intubation (N=6,885).**

| | **Full sample** | **Cox regression adjusted for age and sex** | **Multivariable Cox regression analysis** | **Analysis weighted by inverse-probability-weighting weights** | **Matched analytic sample** | **Univariate Cox regression in a matched analytic sample** |
|---|---|---|---|---|---|---|
| | Number of events / Number of patients | HR (CI 95%; p-value) | HR (CI 95%; p-value) | HR (IC95%; p-value) | Number of events / Number of patients (N/%) | HR (CI 95%; p-value) |
| No antidepressant during the hospitalization and in the past 3 months and | 1,188 / 6,885 (17.3%) | Ref. | Ref. | Ref. | Ref. | Ref. |
| | | | | | | |
| Any antidepressant in the past 3 months but not during the hospitalization | 55 / 159 (34.6%) | 0.78 (0.55 -1.05; 0.094) | 1.04 (0.76 -1.43; 0.792) | 0.93 (0.68 - 1.27; 0.644) | 104 / 318 (32.7%) | 0.84 (0.60 -1.17; 0.305) |
| | | | | | | |
| SSRIs in the past 3 months but not during the hospitalization | 31 / 93 (%) | 0.79 (0.51-1.22; 0.281) | 1.07 (0.69 - 1.67; 0.746) | 0.82 (0.55 - 1.21; 0.318) | 62 / 186 (%) | 0.75 (0.49 -1.16; 0.185) |

| | | | | | | |
|---|---|---|---|---|---|---|
| * p-value is significant (p<0.05). | | | | | | |

**Table 20. Associations of any antidepressant and antidepressant classes with the risks of death and intubation, respectively, in the full sample and in the matched analytic sample.**

| | **Full sample** | **Cox regression adjusted for age and sex** | **Multivariable (Weighted) Cox regression analysis** | **Analysis weighted by inverse-probability-weighting weights** | **Matched analytic sample** | **Univariate Cox regression in a matched analytic sample** |
|---|---|---|---|---|---|---|
| | Number of events / Number of patients (N/%) | HR (CI 95%; p-value) | HR (CI 95%; p-value) | HR (CI 95%; p-value) | Number of events / Number of patients (N / %) | HR (CI 95%; p-value) |
| ***No antidepressant*** | | | | | | |
| *Death* | 866 / 6,885 (12.6%) | Ref. | Ref. | Ref. | Ref. | Ref. |
| *Intubation* | 507 / 6,885 (7.4%) | Ref. | Ref. | Ref. | Ref. | Ref. |

| ***Any antidepressant*** | | | | | | |
|---|---|---|---|---|---|---|
| *Death* | 128 / 460 (27.8%) | 1.02 (0.83 - 1.24; 0.880) | 0.73 (0.59 - 0.90 ; 0.004*) | 0.72 (0.56 - 0.91 ; 0.006*) | 152 / 460 (20.0%) | 0.75 (0.59 - 0.95 ; 0.017*) |
| *Intubation* | 25 / 460 (5.4%) | 0.88 (0.56 -1.39; 0.575) | 0.56 (0.36 - 0.89 ; 0.015*) | 0.59 (0.63 - 0.95 ; 0.030*) | 55 / 460 (%) | 0.42 (0.26 - 0.67 ; <0.001*) |

| ***SSRIs*** | | | | | | |
|---|---|---|---|---|---|---|
| *Death* | 68 / 257 (%) | 0.94 (0.73 -1.22; 0.664) | 0.64 (0.49 - 0.84 ; 0.001*) | 0.65 (0.47 - 0.88; 0.005*) | 89 / 257 (34.6%) | 0.70 (0.50 - 0.93; 0.016*) |
| *Intubation* | 12 / 257 (%) | 0.67 (0.38 - 1.20; 0.182) | 0.39 (0.21- 0.73; 0.003*) | 0.48 (0.25 - 0.91; 0.026*) | 39 / 257 (15.2%) | 0.28 (0.15 - 0.53; <0.001*) |

| ***SNRIs*** | | | | | | |
|---|---|---|---|---|---|---|
| *Death* | 17 / 71 (23.9%) | 0.99 (0.59 - 1.67; 0.979) | 0.66 (0.41 - 1.09; 0.104) | 0.71 (0.42 - 1.23; 0.223) | 21 / 71 (29.6%) | 0.74 (0.39 -1.40; 0.358) |
| *Intubation* | 2 / 71 (2.8%) | 0.35 (0.09 - 1.40; 0.138) | 0.13 (0.02 - 0.95; 0.044*) | 0.29 (0.07 - 1.24; 0.094) | 66 / 71 (8.5%) | 0.32 (0.06 - 1.58; 0.161) |

| ***Tricyclic antidepressants*** | | | | | | |
|---|---|---|---|---|---|---|
| *Death* | 15 / 59 (25.4%) | 1.35 (0.81- 2.24; 0.245) | 0.92 (0.51- 1.65; 0.773) | 1.06 (0.58 - 1.92; 0.851) | 11 / 59 (18.64%) | 1.39 (0.64 - 3.04; 0.404) |
| *Intubation* | 6 / 59 (10.2%) | 1.52 (0.98 - 3.41; 0.311) | 1.88 (0.92 - 3.85; 0.082) | 0.88 (0.35 - 2.24; 0.788) | 11 / 59 (18.6%) | 0.50 (0.18 -1.35; 0.172) |

| ***Tetracyclic antidepressants*** | | | | | | |
|---|---|---|---|---|---|---|
| *Death* | 41 / 94 (46.6%) | 1.38 (0.99 - 1.91; 0.056) | 0.99 (0.70 -1.39; 0.942) | 0.88 (0.61- 1.27; 0.489) | 36 / 94 (38.3%) | 1.12 (0.72 -1.77; 0.613) |
| *Intubation* | 4 / 94 (4.3%) | 0.84 (0.31 - 2.26; 0.733) | 0.38 (0.11-1.35; 0.135) | 1.06 (0.38 - 2.92; 0.913) | 7 / 94 (7.4%) | 0.54 (0.16 -1.84; 0.322) |

| ***Presynaptic α2-antagonist antidepressant*** | | | | | | |
|---|---|---|---|---|---|---|
| *Death* | 11 / 44 (25.0%) | 0.68 (0.34 - 1.26; 0.223) | 0.52 (0.26 - 1.01; 0.055) | 0.54 (0.28 - 1.06; 0.074) | 14 / 44 (31.8%) | 0.69 (0.31 - 1.52; 0.354) |
| *Intubation* | 2 / 44 (4.5%) | 1.07 (0.27 - 4.33; 0.922) | 1.02 (0.26 - 3.93; 0.982) | 1.23 (0.28 - 5.45; 0.790) | 2 / 44 (4.5%) | 0.90 (0.13 - 6.42; 0.916) |

| | | | | | | |
|---|---|---|---|---|---|---|
| * p-value is significant (p<0.05). | | | | | | |

### References

1. Anderson RM, Heesterbeek H, Klinkenberg D, Hollingsworth TD. How will country-based mitigation measures influence the course of the COVID-19 epidemic? Lancet. Mar 21 2020;395(10228):931-934.
2. Beigel JH, Tomashek KM, Dodd LE, et al. Remdesivir for the Treatment of Covid-19 - Preliminary Report. New England Journal of Medicine. 2020.
3. Gordon DE, Jang GM, Bouhaddou M, et al. A SARS-CoV-2 protein interaction map reveals targets for drug repurposing. Nature. 2020:1-13.
4. Chevance A, Gourion D, Hoertel N, et al. Ensuring mental health care during the SARS-CoV-2 epidemic in France: a narrative review. L'encephale. 2020.
5. Stebbing J, Phelan A, Griffin I, et al. COVID-19: combining antiviral and anti-inflammatory treatments. The Lancet Infectious Diseases. 2020;20(4):400-402.
6. Liu Y, Yan L-M, Wan L, et al. Viral dynamics in mild and severe cases of COVID-19. The Lancet Infectious Diseases. 2020.
7. Köhler CA, Freitas TH, Stubbs B, et al. Peripheral alterations in cytokine and chemokine levels after antidepressant drug treatment for major depressive disorder: systematic review and meta-analysis. Molecular neurobiology. 2018;55(5):4195-4206.
8. Hiles S, Baker A, de Malmanche T, Attia J. Interleukin-6, C-reactive protein and interleukin-10 after antidepressant treatment in people with depression: a meta-analysis. Psychological medicine. 2012;42(10):2015-2026.
9. Ye Q, Wang B, Mao J. The pathogenesis and treatment of theCytokine Storm'in COVID-19. Journal of infection. 2020;80(6):607-613.
10. Tynan RJ, Weidenhofer J, Hinwood M, Cairns MJ, Day TA, Walker FR. A comparative examination of the anti-inflammatory effects of SSRI and SNRI antidepressants on LPS stimulated microglia. Brain, behavior, and immunity. 2012;26(3):469-479.
11. Zimniak M, Kirschner L, Hilpert H, Seibel J, Bodem J. The serotonin reuptake inhibitor Fluoxetine inhibits SARS-CoV-2. bioRxiv. 2020:2020.2006.2014.150490.
12. Tham A, Jonsson U, Andersson G, Söderlund A, Allard P, Bertilsson G. Efficacy and tolerability of antidepressants in people aged 65 years or older with major depressive disorder-A systematic review and a meta-analysis. Journal of affective disorders. 2016;205:1-12.
13. Krause M, Gutsmiedl K, Bighelli I, Schneider-Thoma J, Chaimani A, Leucht S. Efficacy and tolerability of pharmacological and non-pharmacological interventions in older patients with major depressive disorder: A systematic review, pairwise and network meta-analysis. European Neuropsychopharmacology. 2019.
14. Cipriani A, Furukawa TA, Salanti G, et al. Comparative efficacy and acceptability of 21 antidepressant drugs for the acute treatment of adults with major depressive disorder: a systematic review and network meta-analysis. Focus. 2018;16(4):420-429.
15. Williamson E, Walker AJ, Bhaskaran KJ, et al. OpenSAFELY: factors associated with COVID-19-related hospital death in the linked electronic health records of 17 million adult NHS patients. MedRxiv. 2020.
16. Zhou F, Yu T, Du R, et al. Clinical course and risk factors for mortality of adult inpatients with COVID-19 in Wuhan, China: a retrospective cohort study. The Lancet. 2020.
17. Ruan Q, Yang K, Wang W, Jiang L, Song J. Clinical predictors of mortality due to COVID-19 based on an analysis of data of 150 patients from Wuhan, China. Intensive care medicine. 2020:1-3.
18. Hur K, Price CP, Gray EL, et al. Factors Associated With Intubation and Prolonged Intubation in Hospitalized Patients With COVID-19. Otolaryngology--Head and Neck Surgery*.*
19. Haut Conseil de la Santé Publique. Statement on the management at home or in a care facility of suspected or confirmed Covid-19 patients. April 8, 2020. Statement on the management at home or in a care facility of suspected or confirmed Covid-19 patients. <https://Erreur ! Référence de lien hypertexte non vali**de.**
20. Lagunas-Rangel FA. Neutrophil-to-lymphocyte ratio and lymphocyte-to-C-reactive protein ratio in patients with severe coronavirus disease 2019 (COVID-19): A meta-analysis. Journal of medical virology. 2020.
21. Devlin J, Chang M-W, Lee K, Toutanova K. Bert: Pre-training of deep bidirectional transformers for language understanding. arXiv preprint arXiv:1810.04805. 2018.
22. Jouffroy J, Feldman SF, Lerner I, Rance B, Neuraz A, Burgun A. MedExt: combining expert knowledge and deep learning for medication extraction from French clinical texts.
23. Robins JM, Hernan MA, Brumback B. Marginal structural models and causal inference in epidemiology. LWW; 2000.
24. Geleris J, Sun Y, Platt J, et al. Observational study of hydroxychloroquine in hospitalized patients with Covid-19. New England Journal of Medicine. 2020.
25. Kaplan EL, Meier P. Nonparametric estimation from incomplete observations. Journal of the American statistical association. 1958;53(282):457-481.
26. Efron B. Nonparametric standard errors and confidence intervals. canadian Journal of Statistics. 1981;9(2):139-158.
27. Hayasaka Y, Purgato M, Magni LR, et al. Dose equivalents of antidepressants: Evidence-based recommendations from randomized controlled trials. Journal of affective disorders. Jul 15 2015;180:179-184.
28. Von Elm E, Altman DG, Egger M, Pocock SJ, Gøtzsche PC, Vandenbroucke JP. The Strengthening the Reporting of Observational Studies in Epidemiology (STROBE) statement: guidelines for reporting observational studies. Annals of internal medicine. 2007;147(8):573-577.

## Claims

1. A serotonin receptor (5 HT R)-stimulating or blocking agent for use for treating a viral infection due to at least one betacoronavirus in a patient in need thereof.

2. The 5 HTR-stimulating or blocking agent for use according to claim 1, wherein the betacoronavirus is a Severe Acute Respiratory Syndrome-related coronavirus (SARS-CoV), in particular SARS-CoV-2.

3. The 5 HTR-stimulating or blocking agent for use according to claim 2, for treating patients suffering from a symptomatic COVID-19 disease, and advantageously from a strong or severe symptomatic COVID-19 disease.

4. The 5 HTR-stimulating or blocking agent for use according to any one of claims 1 to 3 for preventing and/or treating acute respiratory distress syndrome associated to said viral infection.

5. The 5 HTR-stimulating or blocking agent for use according to any one of claims 1 to 4 for lowering the risk of intubation or for increasing the survival rate of patients infected by said betacoronavirus.

6. The 5 HTR-stimulating or blocking agent for use according to any one of claims 1 to 5, said agent being selected in the group consisting of: serotonin reuptake inhibitors (SRIs); tricyclic antidepressants (TCAs); and monoamine oxidase inhibitors (MAOs); noradrenergic and specific serotoninergic antidepressants (NaSSAs); atypical antidepressants and antidepressant enhancers.

7. The 5 HTR-stimulating or blocking agent for use according to claim 6, wherein said serotonin reuptake inhibitor (SRI) is chosen in the group consisting of: selective serotonin reuptake inhibitors (SSRIs), serotonin and norepinephrine reuptake inhibitors (SNRIs), serotonin-norepinephrine-dopamine reuptake inhibitor (SNDRIs) or metabolites thereof.

8. The 5 HTR-stimulating or blocking agent for use according to claim 7, wherein said SSRI is selected from the group consisting of: fluoxetine, citalopram, escitalopram, sertraline, norsertraline, paroxetine, fluvoxamine, femoxetine, indalpine, alaproclate, cericlamine, ifoxetine, zimelidine dapoxetine, and etoperidone, vortioxetine or a pharmaceutical salt thereof.

9. The 5 HTR-stimulating or blocking agent for use according to claim 7, wherein said SNRI is selected from the group consisting of: duloxetine, venlafaxine, desvenlafaxine, milnacipran, levominalcipran, and sibutramine or a pharmaceutical salt thereof.

10. The 5 HTR-stimulating or blocking agent for use according to claim 6, wherein it blocks presynaptic alpha-2 adrenergic receptors, and is selected in the group consisting of: mirtazapine, aptazapine, esmirtazapine, setiptiline and S32212 (also known as N-[4-methoxy-3-(4-methylpiperazin-1-yl)phenyl]-1,2-dihydro-3H-benzo[e]indole-3-carbo-xamide), or a pharmaceutical salt thereof.

11. The 5 HTR-stimulating or blocking agent for use according to any of claim 1-10, wherein it is chosen in the group consisting of: duloxetine, venlafaxine, mirtazapine, fluoxetine, citalopram, escitalopram, sertraline and paroxetine.

12. The 5 HTR-stimulating or blocking agent for use according to any of claim 1 to 11, wherein it is administered at an effective Fluoxetine-equivalent dose comprised between about 5 mg/day and about 60 mg/day, preferably between about 20 mg/day and about 40 mg/day.
